(19) Europäisches Patentamt
European Patent Office
Office européen des brevets

(11) Veröffentlichungsnummer: **0 280 873**
**A1**

(12) **EUROPÄISCHE PATENTANMELDUNG**

(21) Anmeldenummer: **88101153.0**

(22) Anmeldetag: **27.01.88**

(51) Int. Cl.⁴: **C07D 417/04**, A61K 31/425, A61K 31/445

(30) Priorität: **05.02.87 DE 3703435**

(43) Veröffentlichungstag der Anmeldung: **07.09.88 Patentblatt 88/36**

(84) Benannte Vertragsstaaten: **AT BE CH DE ES FR GB GR IT LI LU NL SE**

(71) Anmelder: **Dr. Karl Thomae GmbH**
**Postfach 1755**
**D-7950 Biberach 1(DE)**

(72) Erfinder: **Hurnaus, Rudolf, Dr. Dipl.-Chem.**
**Silcherstrasse 19**
**D-7950 Biberach 1(DE)**
Erfinder: **Sauter, Robert, Dr. Dipl.-Chem.**
**Albert-Schweitzer-Weg 9**
**D-7958 Laupheim(DE)**
Erfinder: **Reiffen, Manfred, Dr. Dipl.-Chem.**
**Matthias-Erzberger-Strasse 40**
**D-7950 Biberach 1(DE)**
Erfinder: **Grell, Wolfgang, Dr. Dipl.-Chem.**
**Amriswilstrasse 7**
**D-7950 Biberach 1(DE)**
Erfinder: **Kobinger, Walter, Prof. Dr.**
**Beighofergasse 27**
**A-1121 Wien(AT)**
Erfinder: **Pichler, Ludwig, Dr.**
**Gusshausstrasse 24/11**
**A-1010 Wien(AT)**
Erfinder: **Schingnitz, Günter, Dr.**
**Unter den Gärten 18**
**D-6550 Bad Kreuznach(DE)**
Erfinder: **Entzeroth, Michael, Dr. Dipl.-Chem.**
**Riedbeundweg 11**
**D-7950 Biberach 19(DE)**

(54) **Neue Piperidine, diese Verbindungen enthaltende Arzneimittel und Verfahren zu ihrer Herstellung.**

(57) Die vorliegende Erfindung betrifft neue Piperidine der allgemeinen Formel

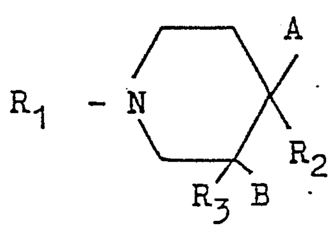

,(I)

**EP 0 280 873 A1**

in der

A und B jeweils ein Wasserstoffatom oder zusammen eine weitere Kohlenstoff-Kohlenstoff-Bindung,

$R_1$ ein Wasserstoffatom, eine gegebenenfalls durch eine Phenylgruppe substituierte Alkylgruppe, eine gegebenenfalls durch eine Phenylgruppe substituierte Alkenyl-oder Alkinylgruppe,

einer der Reste $R_2$ oder $R_3$ eine Gruppe der Formel

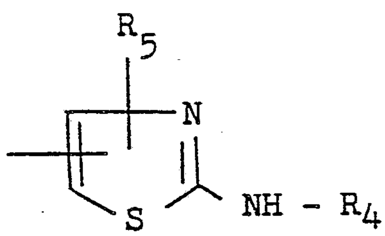

und der andere der Reste $R_2$ oder $R_3$ ein Wasserstoffatom, wobei $R_4$ ein Wasserstoffatom, eine gegebenenfalls durch eine Phenylgruppe substituierte Alkylgruppe oder eine gegebenenfalls durch eine Phenylgruppe substituierte Alkanoylgruppe, wobei die vorstehend bei der Definition der Reste $R_1$ und $R_4$ erwähnten Phenylkerne jeweils durch ein Halogenatom, eine Methyl-oder Methoxygruppe substituiert sein können, und $R_5$ ein Wasserstoffatom oder eine Alkylgruppe darstellen, deren Enantiomere und deren Säureadditionssalze.

Die neuen Verbindungen weisen wertvolle pharmakologische Eigenschaften auf, nämlich eine herzfrequenzsenkende Wirkung und eine Wirkung auf das Zentralnervensystem, insbesondere jedoch eine dopaminerge Wirkung, und lassen sich nach an und für sich bekannten Verfahren herstellen.

## Neue Piperidine, diese Verbindungen enthaltende Arzneimittel und Verfahren zu ihrer Herstellung

Gegenstand der vorliegenden Erfindung sind neue Piperidine der allgemeinen Formel

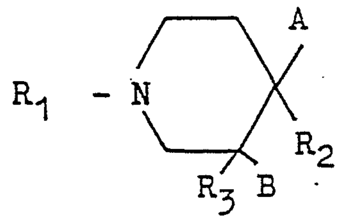
,(I)

deren Enantiomere, falls diese ein optisch aktives Kohlenstoffatom enthalten, und die Säureadditionssalze dieser Verbindungen, insbesondere für die pharmazeutische Anwendung, deren physiologisch verträgliche Säureadditionssalze mit anorganischen oder organischen Säuren, und Verfahren zu ihrer Herstellung.

Die neuen Verbindungen weisen wertvolle pharmakologische Eigenschaften auf, nämlich eine herzfrequenzsenkende Wirkung und eine Wirkung auf das Zentralnervensystem, insbesondere jedoch eine Wirkung auf das dopaminerge System.

In der obigen allgemeinen Formel bedeuten

A und B jeweils ein Wasserstoffatom oder zusammen eine weitere Kohlenstoff-Kohlenstoff-Bindung,

$R_1$ ein Wasserstoffatom, eine gegebenenfalls durch eine Phenylgruppe substituierte Alkylgruppe mit 1 bis 3 Kohlenstoffatomen, eine Alkylgruppe mit 4 bis 6 Kohlenstoffatomen, eine gegebenenfalls durch eine Phenylgruppe substituierte Alkenyl-oder Alkinylgruppe mit jeweils 3 bis 5 Kohlenstoffatomen,

einer der Reste $R_2$ oder $R_3$ eine Gruppe der Formel

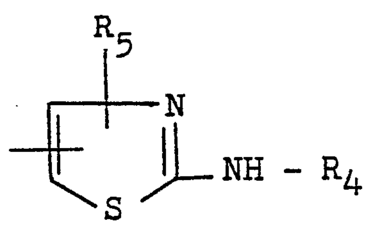

und der andere der Reste $R_2$ oder $R_3$ ein Wasserstoffatom, wobei $R_4$ ein Wasserstoffatom, eine gegebenenfalls durch eine Phenylgruppe substituierte Alkylgruppe mit 1 bis 3 Kohlenstoffatomen oder eine gegebenenfalls durch eine Phenylgruppe substituierte Alkanoylgruppe mit 1 bis 4 Kohlenstoffatomen, wobei die vorstehend bei der Definition der Reste $R_1$ und $R_4$ erwähnten Phenylkerne jeweils durch ein Halogenatom, eine Methyl-oder Methoxygruppe substituiert sein können, und

$R_5$ ein Wasserstoffatom oder eine Alkylgruppe mit 1 bis 3 Kohlenstoffatomen darstellen.

Für die bei der Definition der eingangs erwähnten Reste kommt beispielsweise für die

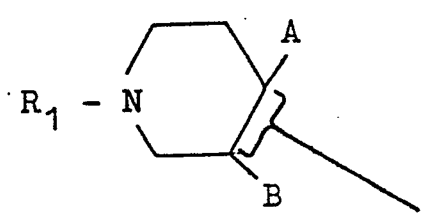

Gruppe die Bedeutung der Piperidin-3-yl-, Piperidin-4-yl-, 1,2,3,6-Tetrahydro-pyridin-5-yl-, 1,2,3,6-Tetrahydro-pyridin-4-yl-, 1-Methyl-piperidin-3-yl-, 1-Methyl-piperidin-4-yl-, 1-Methyl-1,2,3,6-tetrahydro-pyridin-5-yl-, 1-Methyl-1,2,3,6-tetrahydro-pyridin-4-yl-, 1-Ethyl-piperidin-3-yl-, 1-Ethyl-piperidin-4-yl-, 1-Ethyl-1,2,3,6-tetrahydro-pyridin-5-yl-, 1-Ethyl-1,2,3,6-tetrahydro-pyridin-4-yl-, 1-n-Propyl-piperidin-3-yl-, 1-n-Propyl-piperidin-4-yl-, 1-n-Propyl-1,2,3,6-tetrahydro-pyridin-5-yl-, 1-n-Propyl-1,2,3,6-tetrahydro-pyridin-4-yl-, 1-Isopropyl-piperidin-3-yl-, 1-Isopropyl-piperidin-4-yl-, 1-Isopropyl-1,2,3,6-tetrahydro-pyridin-5-yl-, 1-Isopropyl-1,2,3,6-tetrahydro-pyridin-4-yl-, 1-n-Butyl-piperidin-3-yl-, 1-n-Butyl-piperidin-4-yl-, 1-n-Butyl-1,2,3,6-tetrahydro-pyridin-5-yl-, 1-n-Butyl-1,2,3,6-tetrahydro-pyridin-4-yl-, 1-Isobutyl-piperidin-3-yl-, 1-Isobutyl-piperidin-4-yl-, 1-Isobutyl-1,2,3,6-tetrahydro-pyridin-5-yl-, 1-Isobutyl-1,2,3,6-tetrahydro-pyridin-4-yl-, 1-n-Pentyl-piperidin-3-yl-, 1-n-Pentyl-piperidin-4-yl-, 1-n-Pentyl-1,2,3,6-tetrahydro-pyridin-5-yl-, 1-n-Pentyl-1,2,3,6-tetrahydro-pyridin-4-yl-, 1-n-Hexyl-piperidin-3-yl-, 1-n-Hexyl-piperidin-4-yl-, 1-n-Hexyl-1,2,3,6-tetrahydro-pyridin-4-yl-, 1-n-Hexyl-1,2,3,6-tetrahydro-pyridin-5-yl-, 1-Benzyl-piperidin-3-yl-, 1-benzyl-piperidin-4-yl-, 1-Benzyl-1,2,3,6-tetrahydropyridin-5-yl-, 1-Benzyl-1,2,3,6-tetrahydro-pyridin-4-yl-, 1-(4-Fluorbenzyl)-piperidin-3-yl-, 1-(4-Fluorbenzyl)-piperidin-4-yl-, 1-(4-Fluorbenzyl)-1,2,3,6-tetrahydro-pyridin-5-yl-, 1-(4-Fluorbenzyl)-1,2,3,6-tetrahydro-pyridin4-yl-, 1-(4-Chlorbenzyl)-piperidin-3-yl-, 1-(4-Chlorbenzyl)-piperidin-4-yl-, 1-(4-Chlorbenzyl)-1,2,3,6-tetrahydro pyridin-5-yl-, 1-(4-Chlorbenzyl)-1,2,3,6-tetrahydro-pyridin-4-yl-, 1-Cinnamyl-piperidin-3-yl-, 1-Cinnamyl-piperidin-4-yl-, 1-Cinnamyl-1,2,3,6-tetrahydro-pyridin-5-yl-, 1-Cinnamyl-1,2,3,6-tetrahydro-pyridin-4-yl-, 1-(3-Fluorcinnamyl)-piperidin-3-yl-, 1-(3-Fluor-cinnamyl)-piperidin-4-yl-, 1-(3-Fluor-cinnamyl) 1,2,3,6-tetrahydro-pyridin-4-yl-, 1-(3-Fluor-cinnamyl)-1,2,3,6-tetrahydro-pyridin-5-yl-, 1-(3-Chlor-cinnamyl)-piperidin-3-yl-, 1-(3-Chlorcinnamyl)-piperidin-4-yl-, 1-(3-Chlor-cinnamyl) 1,2,3,6-tetrahydro-pyridin-4-yl-, 1-(3-Chlor-cinnamyl)-1,2,3,6-tetrahydro-pyridin-5-yl-, 1-(3-Methyl-cinnamyl)-piperidin-3-yl-, 1-(3-Methyl-cinnamyl)-piperidin-4-yl-, 1-(3-Methyl-cinnamyl)-1,2,3,6-tetrahydro-pyridin-4-yl-, 1-(3-Methylcinnamyl) 1,2,3,6-tetrahydro-pyridin-5-yl-, 1-(3-Methoxycinnamyl)-piperidin-3-yl-, 1-(3-Methoxy-cinnamyl)-piperidin-4-yl-, 1-(3-Methoxy-cinnamyl)-1,2,3,6-tetrahydro-pyridin-4-yl-, 1-(3-Methoxy-cinnamyl)-1,2,3,6-tetrahydro-pyridin-5-yl-, 1-(4-Brombenzyl)-piperidin-3-yl-, 1-(4-Brombenzyl)-piperidin-4-yl-, 1-(4-Brombenzyl) 1,2,3,6-tetrahydropyridin-5-yl-, 1-(4-Brombenzyl)-1,2,3,6-tetrahydro-pyridin-4-yl-, 1-(4-Methylbenzyl)-piperidin-3-yl-, 1-(4-Methylbenzyl)-piperidin-4-yl-, 1-(4-Methylbenzyl) 1,2,3,6-tetrahydro-pyridin-5-yl-, 1-(4-Methylbenzyl) 1,2,3,6 tetrahydropyridin-4-yl-, 1-(4-Methoxybenzyl) piperidin-3-yl-, 1-(4-Methoxybenzyl)-piperidin-4-yl-, 1-(4-Methoxybenzyl)-1,2,3,6-tetrahydro-pyridin-5-yl-, 1-(4-Methoxybenzyl)-1,2,3,6-tetrahydro-pyridin-4-yl-, 1-(1-Phenylethyl)-piperidin-3-yl-, 1-(1-Phenylethyl)-piperidin-4-yl-, 1-(1-Phenylethyl)-1,2,3,6-tetrahydro-pyridin-5-yl-, 1-(1-Phenylethyl)-1,2,3,6-tetrahydro-pyridin-4-yl-, 1-(2-Phenylethyl)-piperidin-3-yl-, 1-(2-Phenylethyl)-piperidin-4-yl-, 1-(2-Phenylethyl)-1,2,3,6-tetrahydro-pyridin-5-yl-, 1-(2-Phenylethyl)-1,2,3,6-tetrahydro-pyridin-4-yl-, 1-(3-Phenyl-n-propyl)-piperidin-3-yl-, 1-(3-Phenyl-n-propyl)-piperidin-4-yl-, 1-(3-Phenyl-n-propyl)-1,2,3,6-tetrahydro-pyridin-5-yl-, 1-(3-Phenyl-n-propyl-1,2,3,6-tetrahydro-pyridin-4-yl-, 1-Allyl-piperidin-3-yl-, 1-Allyl-piperidin-4-yl-, 1-Allyl-1,2,3,6-tetrahydro-pyridin-5-yl-, 1-Allyl-1,2,3,6-tetrahydro-pyridin-4-yl-, 1-(2-Buten-1-yl)-piperidin-3-yl-, 1-(2-Buten-1-yl)-piperidin-4-yl-, 1-(2-Buten-1-yl)-1,2,3,6-tetrahydro-pyridin-5-yl-, 1-(2-Buten-1-yl)-1,2,3,6-tetrahydro-pyridin-4-yl-, 1-(2-Penten-1-yl)-piperidin-3-yl-, 1-(2-Penten-1-yl)-piperidin-4-yl-, 1-(2-Penten-1-yl) 1,2,3,6-tetrahydro-pyridin-5-yl-, 1-(2-Penten-1-yl) 1,2,3,6-tetrahydro-pyridin-4-yl-, 1-(3-Buten-2-yl)-piperidin-3-yl-, 1-(3-Buten-2-yl)-piperidin-4-yl-, 1-(3-Buten-2-yl) 1,2,3,6-tetrahydro-pyridin-5-yl-, 1-(3-Buten-2-yl)-1,2,3,6-tetrahydro-pyridin-4-yl-, 1-(2-Methyl-allyl)-piperidin-3-yl-, 1-(2-Methyl-allyl)-piperidin-4-yl-, 1-(2-Methyl-allyl)-1,2,3,6-tetrahydro-pyridin-5-yl-, 1-(2-Methyl-allyl)-1,2,3,6-tetrahydro-pyridin-4-yl-, 1-Propargyl-piperidin-3-yl-, 1-Propargyl-piperidin-4-yl-, 1-Propargyl-1,2,3,6-tetrahydro-pyridin-5-yl-, 1-Propargyl-1,2,3,6-tetrahydropyridin-4-yl-, 1-(2-Butin-1-yl)-piperidin-3-yl-, 1-(2-Butin-1-yl)-piperidin-4-yl-, 1-(2-Butin-1-yl)-1,2,3,6-tetrahydro-pyridin-5-yl-, 1-(2-Butin-1-yl)-1,2,3,6-tetrahydropyridin-4-yl-, 1-(2-Pentin-1-yl)-piperidin-3-yl-, 1-(2-Pentin-1-yl)-piperidin-4-yl-, 1-(2-Pentin-1-yl)-1,2,3,6-tetrahydro-pyridin-5-yl-, 1-(2-Pentin-1-yl)-1,2,3,6-tetrahydro-pyridin-4-yl-, 1-(3-Butin-2-yl) piperidin-3-yl-, 1-(3-Butin-2-yl)-piperidin-4-yl-, 1-(3-Butin-2-yl)-1,2,3,6-tetrahydro-pyridin-5-yl-und 1-(3-Butin-2-yl)-1,2,3,6-tetrahydro-pyridin4-yl-gruppe,

für $R_4$ die des Wasserstoffatoms, der Methyl-, Ethyl-, n-propyl-, Isopropyl-, Benzyl-, Chlorbenzyl-, Methoxybenzyl-, Methylbenzyl-, 1-Phenylethyl-, 2-Phenylethyl-, 3-Phenylpropyl-, Formyl-, Acetyl-, Propionyl-, Butanoyl-, Benzoyl-, Chlorbenzoyl-, Methoxybenzoyl-, Methylbenzoyl-, Phenacetyl-oder Phenylpropionylgruppe und

für $R_5$ die des Wasserstoffatoms, der Methyl-, Ethyl-oder n-Propylgruppe in Betracht.

Beispielsweise seien folgende Verbindungen erwähnt, die unter die vorstehende allgemeine Formel I fallen, aber nicht in den Beispielen ausdrücklich beschrieben werden:

2-Amino-4-(1-allyl-piperidin-3-yl)thiazol,
2-Amino-4-(1-allyl-piperidin-4-yl)thiazol,
2-Amino-4-(1-butyl-1,2,3,6-tetrahydro-pyridin-4-yl)thiazol,
2-Amino-4-(1-butyl-piperidin-4-yl)thiazol,
2-Amino-4-[1-(2-Phenyl-ethyl)-1,2,3,6-tetrahydro-pyridin-4-yl]thiazol,
2-Amino-4-(1-cinnamyl-piperidin-4-yl)thiazol,
2-Amino-4-(1-cinnamyl-piperidin-3-yl)thiazol,
2-Amino-4-(1-propargyl-1,2,3,6-tetrahydro-pyridin-4-yl)thiazol,
2-Amino-4-(1-propargyl-1,2,3,6-tetrahydro-pyridin-5-yl)thiazol,

2-Amino-4-(1-propargyl-piperidin-4-yl)thiazol,
2-Amino-4-(piperidin-4-yl)thiazol,
2-Amino-4-(piperidin-3-yl)thiazol und
2-Amino-4-(1,2,3,6-tetrahydro-pyridin-4-yl)thiazol
sowie deren Säureadditionssalze, insbesondere deren physiologisch verträgliche Säureadditionssalze.

Bevorzugte Verbindungen der allgemeinen Formel I sind diejenigen, in denen
A, B und $R_1$ wie eingangs definiert sind,
$R_2$ eine Gruppe der Formel

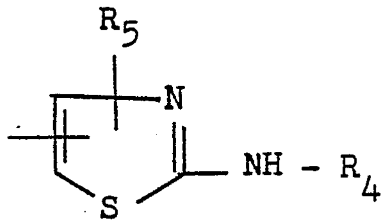

und $R_3$ ein Wasserstoffatom oder
$R_3$ eine Gruppe der Formel

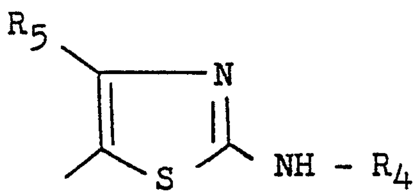

oder auch,
wenn $R_1$ ein Wasserstoffatom, eine durch eine Phenylgruppe substituierte Alkenyl-oder Alkinylgruppe mit jeweils 3 bis 5 Kohlenstoffatomen oder auch eine Alkylgruppe mit 1 bis 6 Kohlenstoffatomen, falls $R_4$ eine durch eine Phenylgruppe substituierte Alkylgruppe mit 1 bis 3 Kohlenstoffatomen oder eine durch eine Phenylgruppe substituierte Alkanoylgruppe mit 1 bis 4 Kohlenstoffatomen darstellt,
eine Gruppe der Formel

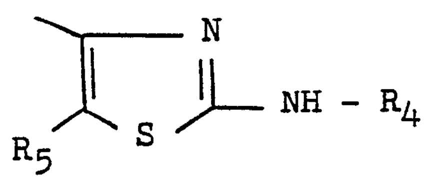

und $R_2$ ein Wasserstoffatom, wobei $R_4$ ein Wasserstoffatom, eine gegebenenfalls durch eine Phenylgruppe substitu ierte Alkylgruppe mit 1 bis 3 Kohlenstoffatomen oder eine gegebenenfalls durch eine Phenylgruppe substituierte Alkanoylgruppe mit 1 bis 4 Kohlenstoffatomen und
$R_5$ ein Wasserstoffatom oder eine Alkylgruppe mit 1 bis 3 Kohlenstoffatomen darstellen,
wobei zusätzlich die bei der Definition der Reste $R_1$ und $R_4$ vorstehend erwähnten Phenylkerne jeweils durch ein Halogenatom, eine Methyl-oder Methoxygruppe substituiert sein können, insbesondere diejenigen Verbindungen der allgemeinen Formel I, in der
A und B wie eingangs definiert sind,
$R_1$ ein Wasserstoffatom, eine Alkylgruppe mit 1 bis 5 Kohlenstoffatomen, eine Allyl-, Cinnamyl-, Methoxycinnamyl-, Benzyl-oder Chlorbenzylgruppe,
$R_2$ eine Gruppe der Formel

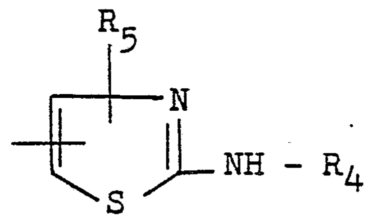

und $R_3$ ein Wasserstoffatom oder auch wenn $R_1$ ein Wasserstoffatom, eine Cinnamyl-oder Methoxycinnamylgruppe darstellt, $R_3$ eine Gruppe der Formel

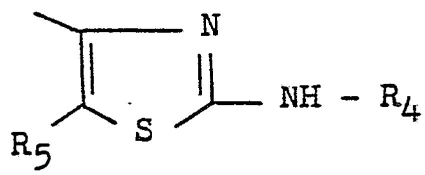

und $R_2$ ein Wasserstoffatom, wobei $R_4$ ein Wasserstoffatom, eine Chlorbenzylgruppe oder eine Alkanoylgruppe mit 2 oder 3 Kohlenstoffatomen und $R_5$ ein Wasserstoffatom oder eine Methylgruppe darstellen, und deren Säureadditionssalze, insbesondere deren physiologisch verträgliche Säureadditionssalze.

Erfindungsgemäß erhält man die neuen Verbindungen nach folgenden Verfahren:

a.) Zur Herstellung von Verbindungen der allgemeinen Formel I, in der A und B zusammen eine weitere Kohlenstoff-Kohlenstoff-Bindung darstellen:

Reduktion einer Pyridiniumverbindung der allgemeinen Formel

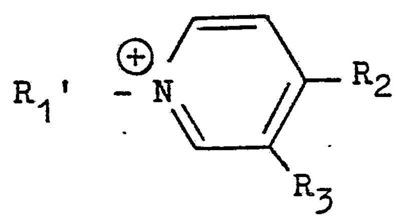

,(II)

in der
$R_2$ und $R_3$ wie eingangs definiert sind,
$R_1'$ mit Ausnahme des Wasserstoffatoms die für $R_1$ eingangs erwähnten Bedeutungen besitzt, mit einem komplexen Metallhydrid.

Die Reduktion wird mit einem geeigneten Metallhydrid wie Natriumborhydrid in einem geeigneten Lösungsmittel wie Methanol, Ethanol oder Ethanol/Wasser bei Temperaturen zwischen 0 und 50°C, vorzugsweise bei Raumtemperatur, durchgeführt.

Eine so erhaltene Verbindung der allgemeinen Formel I, in der $R_1$ einen Benzylrest darstellt, kann nach Überführung in einen entsprechenden Ameisensäureesterrest mittels eines entsprechenden Halogenameisensäureesters, mittels Hydrolyse in eine entsprechende Verbindung der allgemeinen Formel I, in der $R_1$ ein Wasserstoffatom darstellt, übergeführt werden.

Die anschließende Hydrolyse erfolgt vorzugsweise in einem geeigneten Lösungsmittel wie z.B. Wasser, Isopropanol/Wasser, Tetrahydrofuran/Wasser, Dioxan/Wasser oder Eisessig, in Gegenwart einer Säure wie Salzsäure, Bromwasserstoffsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches.

Die zur Abspaltung eines Benzylrestes erfordeliche Überführung dieses Restes in einen entsprechenden Ameisensäureesterrest erfolgt vorzugsweise mit Chlorameisensäurebenzylester in Chloroform als Lösungsmittel und bei Raumtemperatur.

b) Zur Herstellung von Verbindungen der allgemeinen Formel I, in der A und B jeweils Wasserstoffatome darstellen:

Katalytische Hydrierung einer Verbindung der allgemeinen Formel

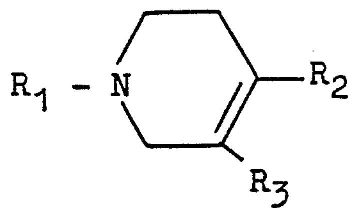

,(III)

in der $R_1$, $R_2$ und $R_3$ wie eingangs definiert sind.

Die katalytische Hydrierung wird mit Wasserstoff in Gegenwart eines Hydrierungskatalysators wie Palladium/Kohle in einem geeigneten Lösungsmittel wie Methanol, Ethanol, Essig säureethylester oder Eisessig bei Temperaturen zwischen 0 und 80°C, vorzugsweise bei Temperaturen zwischen 20 und 60°C, und einem Wasserstoffdruck von 1 bis 7 bar, vorzugsweise von 3 bis 5 bar, durchgeführt, wobei eine im Rest $R_1$ vorhandene Doppel-oder Dreifachbindung gleichzeitig aufhydriert werden kann oder, falls $R_1$ und/oder $R_4$ eine Benzylgruppe bedeuten, diese gleichzeitig abgespalten werden kann.

c) Zur Herstellung von Verbindungen der allgemeinen Formel I, in der $R_1$ und oder $R_4$ ein Wasserstoffatom darstellen:

Abspaltung eines oder zweier Schutzreste von einer Verbindung der allgemeinen Formel

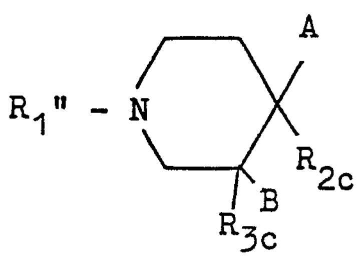

,(IV)

in der

A und B wie eingangs definiert sind,

$R_1''$ einen hydrolytisch abspaltbaren Rest oder die für $R_1$ eingangs erwähnten Bedeutungen besitzt, einer der Reste $R_{2c}$ oder $R_{3c}$ eine Gruppe der Formel

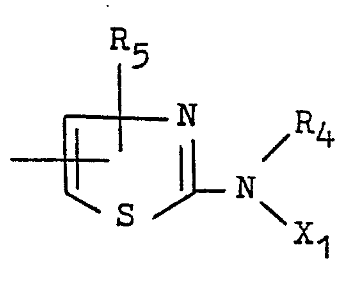

,

wobei $R_5$ wie eingangs definiert ist,

$R_4'$ ein Wasserstoffatom oder einen der für $R_4$ eingangs erwähnten Alkyl-oder gegebenenfalls substituierten Phenylalkylreste und

$X_1$ ein Wasserstoffatom, einen hydrolytisch abspaltbaren Rest oder eine gegebenenfalls durch eine Phenylgruppe substituierte Alkanoylgruppe mit 1 bis 4 Kohlenstoffatomen bedeuten, wobei jedoch mindestens einer der Reste $R_1''$ oder $X_1$ einen hydrolytisch abspaltbaren Rest darstellen muß,

und der andere der Reste $R_{2c}$ oder $R_{3c}$ ein Wasserstoffatom bedeuten.

Die hydrolytische Abspaltung erfolgt vorzugsweise in einem geeigneten Lösungsmittel wie Wasser, Methanol/Wasser, Ethanol/Wasser, Isopropanol/Wasser, Tetrahydrofuran/Wasser, Dioxan/Wasser oder Eisessig in Gegenwart einer Säure wie Salzsäure, Bromwasserstoff oder Schwefelsäure oder in Gegenwart einer Base wie Natriumhydroxid oder Kaliumhydroxid bei Temperaturen zwischen 0 und 100°C, vorzugsweise jedoch bei der Siedetemperatur des Reaktionsgemisches.

d) Zur Herstellung von Verbindungen der allgemeinen Formel I, in der $R_1$ mit Ausnahme des Wasserstoffatoms die für $R_1$ eingangs erwähnten Bedeutungen besitzt:

Umsetzung einer Verbindung der allgemeinen Formel

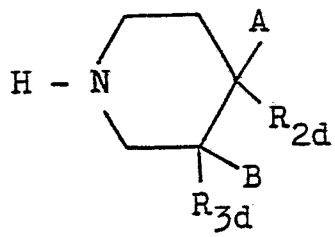

, (V)

in der
A und B wie eingangs definiert sind,
einer der Reste $R_{2d}$ oder $R_{3d}$ eine Gruppe der Formel

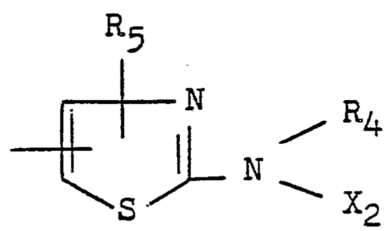

,

wobei $R_4$ und $R_5$ wie eingangs definiert sind und
$X_2$ ein Wasserstoffatom oder einen Schutzrest, falls $R_4$ ein Wasserstoffatom oder einen der für $R_4$ eingangs erwähnten Alkyl-oder gegebenenfalls substituierten Phenylalkylreste darstellt, und der andere der Reste $R_{2d}$ oder $R_{3d}$ein Wasserstoffatom bedeuten, mit einer Verbindung der allgemeinen Formel

$Y_1$ -$R_1'''$ ,(VI)

in der
$R_1'''$ mit Ausnahme des Wasserstoffatoms die für $R_1$ erwähnten Bedeutungen besitzt und
$Y_1$ eine nukleophile Austrittsgruppe oder zusammen mit dem Wasserstoffatom des benachbarten Kohlenstoffatoms ein Sauerstoffatom bedeutet, und gegebenenfalls anschließende Abspaltung eines verwendeten Schutzrestes.

Als Schutzrest für eine Aminogruppe kommen beispielsweise eine Acyl-oder Alkoxycarbonylgruppe wie die Benzoyl-, Methoxycarbonyl-oder Ethoxycarbonylgruppe und
als nukleophile Austrittsgruppe ein Halogenatom wie das Chlor-, Brom-oder Jodatom, eine Sulfonyloxygruppe wie die Methansulfonyloxy-, Methoxy-sulfonyloxy-oder p-Toluolsulfonyloxygruppe in Betracht.

Die Umsetzung wird vorzugsweise in einem Lösungsmittel wie Methanol, Ethanol, Isopropanol, Methylenchlorid, Chloroform, Acetonitril, Toluol, Dioxan oder Dimethylformamid gegebenen falls in Gegenwart eines säurebindenden Mittels wie Kaliumcarbonat, Triethylamin oder Pyridin, oder in Gegenwart eines Reduktionsmittels zweckmäßigerweise bei Temperaturen zwischen 0 und 100°C durchgeführt.

Die Alkylierung wird vorzugsweise mit einer entsprechenden Verbindung der allgemeinen Formel VI, z.B. mit Methyljodid, Ethylbromid, n-Propylbromid, Allylbromid, Dimethylsulfat, Diethylsulfat oder p-Toluolsulfonsäure-ethylester in Gegenwart eines säurebindenden Mittels wie Triethylamin, Kaliumcarbonat oder Pyridin und vorzugsweise bei der Siedetemperatur des Reaktionsgemisches oder mit einer entsprechenden

nichtkonjugierten Carbonylverbindung wie Formalin, Acetaldehyd oder Propionaldehyd in Gegenwart eines Reduktionsmittels wie Ameisensäure oder eines Metallhydrids wie Natriumborhydrid oder Natriumcyanborhydrid, vorzugsweise bei Temperaturen zwischen 20 und 50°C, durchgeführt.

e) Zur Herstellung von Verbindungen der allgemeinen Formel I, in der A und B jeweils ein Wasserstoffatom darstellen:

Umsetzung einer Verbindung der allgemeinen Formel

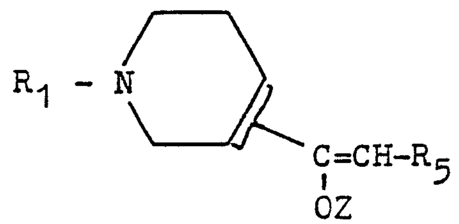

,(VII)

in der
$R_1$ und $R_5$ wie eingangs definiert sind und
Z eine Trialkylsilylgruppe darstellt, mit Brom und anschließend mit einem Thioharnstoff der allgemeinen Formel

$H_2N - CS - NH - R_4$ ,(VIII)

in der
$R_4$ wie eingangs definiert ist.

Die Umsetzung einer Verbindung der allgemeinen Formel VII mit Brom wird vorzugsweise in einem geeigneten Lösungsmittel wie Methylenchlorid, Chloroform oder Eisessig bei Temperaturen zwischen 0 und 50°C, vorzugsweise jedoch bei Raumtemperatur, und die anschließende Umsetzung mit einem Thioharnstoff in der Schmelze oder in einem Lösungsmittel oder Lösungsmittelgemisch wie Wasser, Ethanol, Wasser/Ethanol, Pyridin, Dioxan, Dioxan/Wasser, Eisessig, Tetrahydrofuran oder Dimethylformamid zweckmäßigerweise bei Temperaturen zwischen 0 und 150°C, vorzugsweise bei Temperaturen zwischen 20 und 100°C und gegebenenfalls in Gegenwart einer Base, z.B. Natronlauge, Natriumacetat, Pyridin, Triethylamin oder Ethyldiisopropylamin durchgeführt.

f) Zur Herstellung von Verbindungen der allgemeinen Formel I, in der $R_1$ eine gegebenenfalls durch eine Phenylgruppe substituierte Alkylgruppe mit 1 bis 3 Kohlenstoffatomen oder eine Alkylgruppe mit 4 bis 6 Kohlenstoffatomen und/oder $R_4$ ein Wasserstoffatom oder eine gegebenenfalls durch eine Phenylgruppe substituierte Alkylgruppe mit 1 bis 3 Kohlenstoffatomen bedeuten, wobei die bei der Definition der Reste $R_1$ und $R_4$ vorstehend erwähnten Phenylkerne jeweils durch ein Halogenatom, eine Methyl-oder Methoxygruppe substituiert sein können:

Reduktion einer Verbindung der allgemeinen Formel

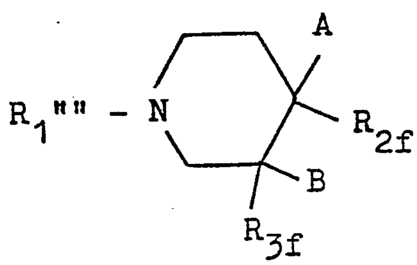

,(IX)

in der
A und B wie eingangs definiert sind,
einer der Reste $R_{2f}$ oder $R_{3f}$ eine Gruppe der Formel

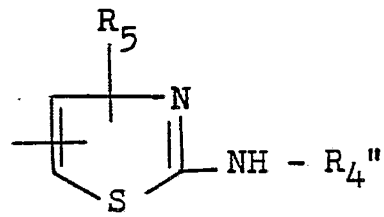

,

wobei $R_5$ wie eingangs definiert ist,
$R_1''''$ und $R_4''$ die für $R_1$ bzw. $R_4$ erwähnten Bedeutungen besitzen, wobei jedoch mindestens in einem der Reste $R_1''''$ oder $R_4''$ die mit dem N-Atom verknüpfte Methylengruppe des Restes $R_1''''$ oder $R_4''$ durch eine Carbonylgruppe ersetzt sein muß, und
der andere der Reste $R_{2f}$ oder $R_{3f}$ ein Wasserstoffatom bedeuten.

Die Reduktion wird in einem geeigneten Lösungsmittel wie Diethylether oder Tetrahydrofuran mit einem Reduktionsmittel wie einem Metallhydrid, z.B. mit Lithiumaluminiumhydrid, Diboran, Diboran/Dimethylsulfid oder mit Natriumborhydrid in Gegenwart von Eisessig oder Trifluoressigsäure, bei Temperaturen zwischen 0 und 50°C, vorzugsweise jedoch bei Temperaturen zwischen 10 und 25°C, durchgeführt.

g) Zur Herstellung von Verbindungen der allgemeinen Formel I, in der $R_4$ eine gegebenenfalls durch eine Phenylgruppe substituierte Alkylgruppe mit 1 bis 3 Kohlenstoffatomen darstellt, wobei der vorstehend erwähnte Phenylkern durch ein Halogenatom, eine Methyl-oder Methoxygruppe substituiert sein kann:

Reduktive Aminierung eines Amins der allgemeinen Formel

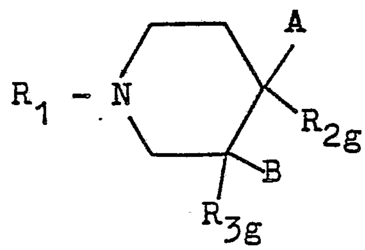

,(X)

in der
A, B und $R_1$ wie eingangs definiert sind,
einer der Reste $R_{2g}$ oder $R_{3g}$ eine Gruppe der Formel

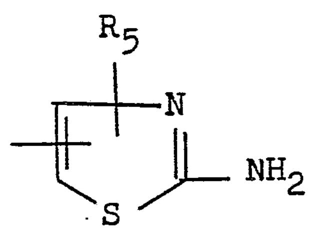

,

wobei $R_5$ wie eingangs definiert ist, und
der andere der Reste $R_{2g}$ oder $R_{3g}$ ein Wasserstoffatom bedeuten, mit einem Aldehyd der allgemeinen Formel

$Y_2$ -$R_4'''$ ,(XI)

in der
$R_4'''$ eine gegebenenfalls durch eine Phenylgruppe substituierte Alkylgruppe mit 1 bis 3 Kohlenstoffatomen darstellt, wobei der vorstehend erwähnte Phenylring durch ein Halogenatom, eine Methyl-oder Methoxygruppe substituiert sein kann, und
$Y_2$ zusammen mit dem Wasserstoffatom des benachbarten Kohlenstoffatoms ein Sauerstoffatom bedeutet.

Die reduktive Aminierung wird in Gegenwart eines Reduktionsmittels wie Ameisensäure oder eines

Metallhydrids wie Natriumborhydrid oder Natriumcyanborhydrid bei Temperaturen zwischen 0 und 50°C, vorzugsweise tei Temperaturen zwischen 20 und 50°C, durchgeführt.

Die erhaltenen Verbindungen der allgemeinen Formel I, welche ein chirales Zentrum enthalten, lassen sich in ihre Enantiomeren nach üblichen Methoden auftrennen, beispielsweise durch Säulenchromatographie an einer chiralen Phase, durch Säulenchromatographie eines Konjugats mit einer optisch aktiven Verbindung und anschließende Spaltung der getrennten Konjugate oder mittels fraktionierter Kristallisation ihrer diastereomeren Salze mit optisch aktiven Hilfssäuren wie Weinsäure, 0,0-Dibenzoylweinsäure, Camphersäure, Camphersulfonsäure oder α-Methoxy-phenylessigsäure.

Ferner lassen sich die erhaltenen Verbindungen in ihre Säureadditionssalze, insbesondere in ihre physiologisch verträglichen Säureadditionssalze mit anorganischen oder organischen Säuren überführen. Als Säuren kommen hierbei beispielsweise Salzsäure, Bromwasserstoffsäure, Schwefelsäure, Phosphorsäure, Milchsäure, Zitronensäure, Weinsäure, Bernsteinsäure, Maleinsäure oder Fumarsäure in Betracht.

Die als Ausgangsstoffe verwendeten Verbindungen der allgemeinen Formeln II bis XI sind teilweise literaturbekannt bzw. man erhält diese nach literaturbekannten Verfahren.

So erhält man beispielsweise eine Verbindung der allgemeinen Formel II durch Ringschluß einer entsprechenden Pyridinverbindung mit einem entsprechenden Thioharnstoff und anschließende Quarternisierung des Pyridinstickstoffes und eine Verbindung der allgemeinen Formel III durch Reduktion einer entsprechenden Pyridiniumverbindung mit einem Metallhydrid.

Zur Herstellung einer Ausgangsverbindung der allgemeinen Formel IV, V, IX oder X wird eine entsprechende Pyridiniumverbindung mit Natriumborhydrid und gegebenenfalls anschließend mit katalytisch angeregtem Wasserstoff reduziert und gegebenenfalls anschließend ein verwendeter Schutzrest abgespalten.

Eine als Ausgangsstoff verwendete Verbindung der allgemeinen Formel VII erhält man durch O-alkylierung eines entsprechenden Ketons, z. B. mit Trimethyl-chlorsilan.

Wie bereits eingangs erwähnt, weisen die neuen Verbindungen und deren physiologisch verträglichen Säureadditionssalze wertvolle pharmakologische Eigenschaften auf, nämlich eine herzfrequenzsenkende Wirkung und eine Wirkung auf das Zentralnervensystem, insbesondere eine dopaminerge Wirkung.

Beispielsweise wurden die Verbindungen

A = 2-Amino-4-(1-propyl-1,2,3,6-tetrahydro-pyridin-5-yl)-thiazol,
B = 2-Amino-4-(1-allyl-1,2,3,6-tetrahydro-pyridin-5-yl)-thiazol,
C = 2-Amino-4-(1-allyl-1,2,3,6-tetrahydro-pyridin-4-yl)-thiazol,
D = 2-Amino-4-(1-propyl-1,2,3,6-tetrahydro-pyridin-4-yl)-thiazol,
E = 2-Amino-4-(1,2,3,6-tetrahydro-pyridin-5-yl)thiazoldihydrochlorid und
F = 2-Amino-4-(1-propyl-piperidin-4-yl)thiazol auf ihre biologischen Eigenschaften wie folgt untersucht:

1.) Bestimmung der Dopamin ($D_2$)-Rezeptorbindungsaffinitäten nach der modifizierten Methode von D.J. de Vries und P.M. Beart (Europ. J. Pharmacology 109, 417-419 (1985)):

Ratten werden durch Schlag betäubt, dekapitiert und das Ge hirn entnommen. Die Striata werden herauspräpariert und in 25 ml eiskaltem Puffer (50 mM TRIS-HCl, 1 mM EDTA, 5 mM $MgCl_2$, 1mM Ascorbinsäure, pH = 7,20) homogenisiert. Das Homogenat wird 15 Minuten bei 0°C und 50000 × g zentrifugiert. Der Überstand wird verworfen. Das Pellet wird durch Resuspension und erneute Zentrifugation zweimal gewaschen und anschließend bei 37°C eine halbe Stunde vorinkubiert. Das Inkubat wird wie zuvor beschrieben zentrifugiert und der Überstand verworfen. Das Pellet wird in 60 ml Puffer resuspen diert, homogenisiert und dann mit Puffer 1:500 bezogen auf das Striatagewicht verdünnt.

Für den Inkubationsansatz werden 10 µl $^3$H-Spiroperidol-Lösung (0,895 Gbq/mMol; Endkonzentration: $2{,}5 \times 10^{-10}$ M), 970 µl Puffer sowie 20 µl Testsubstanzlösung (Endkonzentration: $10^{-11}$-$10^{-4}$ M) vorgelegt. Die unspezifische Bindung wird in Gegenwart von 1 µM Haloperidol bestimmt. Durch Zugabe von 1 ml Homogenatlösung zu dieser Vorlage wird der Assay gestartet. Die Inkubationszeit beträgt 60 Minuten bei Raumtemperatur. Nach Ablauf dieser Zeit wird das Inkubat über ein GF/B-Filter abgesaugt und mit 15 ml eiskaltem Puffer gewaschen. Das feuchte Filter wird mit Instagel® versetzt und über Nacht extrahiert. Anschließend wird die verbliebene Radioaktivität in einem Packard CA 2000 Szintillationscounter bei einer Zählausbeute von 50-55 % gezählt.

Mit Hilfe eines 2-Bindungsstellen-Iterationsprogrammes werden die $IC_{50}$-Werte ermittelt und daraus die $K_i$-Werte berechnet.

| Substanz | $K_i$ [nM] |
|---|---|
| A | 36 |
| B | 8 |
| C | 22 |
| D | 6 |
| E | 3 |
| F | 38 |

2. Bestimmung der Dopaminturnoverhemmung:

Die Dopaminturnoverhemmung wurde an Mäusen gemessen. Bei Tieren, die mit α-Methyl-p-tyrosin (AMPT) (250 mg/kg i.p.) zum Zeitpunkt 15 Minuten des Experimentes behandelt werden, fällt die Dopaminkonzentration im Gesamthirn mit fortschreitender Versuchsdauer ab. Durch Substanzen, die an Dopamin-Autorezeptoren wirken, kann der Dopaminabfall (im Vergleich zu mit Kochsalzlösung behandelten Kontrolltieren) verhindert werden. Testsubstanzen werden zum Zeitpunkt 0 des Experiments subkutan appliziert (5 mg/kg s.c.). Zum Zeitpunkt 4 Stunden und 15 Minuten des Experiments werden die Tiere getötet und die Gehirne der Dopaminbestimmung mittels Hochdruckflüssigkeitschromatographie mit elektrochemischer Detektion zugeführt. Bestimmt wird die durch die Testsubstanz bewirkte prozentuale Hemmung des durch AMPT induzierten Dopaminabfalls:

| Substanz | Hemmung des Dopaminabfalls |
|---|---|
| A | 35 % |
| C | 47 % |
| D | 80 % |
| F | 59 % |

3. Bestimmung der Anti-Parkinson-Wirkung nach der Methode von Hinzen D. et al., European Journal of Pharmacology 131, 75-88 (1986):

a) Motilitätsauslösung an der 24 Stunden zuvor mit Reserpin behandelten Maus:

Mäuse werden 24 Stunden vor dem Experiment mit 5 mg/kg Reserpin i.p. behandelt; die Tiere werden bei 25-30°C gehalten und 3 × mit 2 ml einer 5%igen Glucose-lösung in Tyrode s.c. behandelt. Gruppen zu je 6 Tieren erhalten die Testsubstanz mit 2 mg/kg s.c. injiziert. 20 Minuten später werden die Tiere zur

Aktivitätsmessung in Beobachtungskäfige gesetzt, die mit einer Infrarotlichtschranke ausgerüstet sind. Meßwert ist die Häufigkeit des Passierens des Infrarotstrahles durch eine Gruppe von 6 Mäusen in 5 Minuten ("Laufimpulse/5 Minuten").

Pro Substanz werden 6 Gruppen geprüft. Kontrolltiere erhalten isotone Kochsalzlösung s.c..

Als Ergebnis werden die mit der Testsubstanz erzielten Laufimpulse/5 Minuten dargestellt ($x \pm s_x$) Kontrolltiere (Kochsalzlösung) zeigen minimale Aktivität (ca. 5 Laufimpulse/5 Minuten).

| | Zahl der Laufimpulse/5 min |
|---|---|
| A | 44 ± 4 |
| B | 52 ± 9 |
| C | 32 ± 7 |
| D | 80 ± 10 |
| F | 65 ± 17 |

b) <u>Aufhebung der Parkinsonsymptome am MPTP-Affen nach der modifizierten Methode von Burns, R.S. und Chuang, C.: Proc. Natl. Acad. Sci. 80, 4546-4550 (1983):</u>

Das durch das Neurotoxin 1-Methyl-4-phenyl-1,2,3,6-tetrahydro-pyridin (MPTP) beim Menschen und beim Affen ausgelöste, irreversible neurologische Krankheitsbild ähnelt in seiner klinischen, pathologischen, biochemischen und pharmakologischen Ausprägung weitgehend der idiopathischen Parkinson'schen Erkrankung (Markey et al., Nature 311, 464 (1984)). Ursache dieser überzeugenden Übereinstimmung ist die Tatsache, daß MPTP selektiv jene dopaminergen Nervenzellen in der Substantia nigra des Gehirns zerstört, welche auch bei der natürlich auftretenden Parkinson'schen Erkrankung durch degenerative Prozesse zerstört werden. Es wird diskutiert, daß auch die Ursache der idiopathischen Parkinson'schen Erkrankung im Organismus entstehendes MPTP oder eine ähnliche chemische Verbindung ist (Snyder, S.H., Nature 311, 514 (1984)). Möglicherweise bedingt durch den spezifischen Metabolismus des MPTP ist die klinische Ausprägung des MPTP-Parkinson'bildes bisher außer beim Menschen nur beim Affen nachweisbar.

Das an Rhesusaffen verwirklichte MPTP-Modell ist daher in hervorragendem Maße geeignet, die Wirkung von Anti-Parkinson-Medikamenten zu prüfen. Insgesamt 7 Rhesusaffen waren an dem Versuch beteiligt. Sie hatten über mehrere Tage verteilt, kumulative Dosen von 2,4-6,25 mg/kg MPTP erhalten, bis stabile und irreversible Parkinson-Symptome aufgetreten waren. Im Vordergrund der Symptomatik stand die Bradykinesie, die alle Tiere zeigten, teilweise sogar bis hin zur Akinesie. Kein Tier war in der Lage, spontan Wasser oder Futter aufzunehmen. Die Reaktivität war stark vermindert, meist verharrten die Tiere in einer typischen Beugehaltung. Tremor wurde gelegentlich gesehen. Die Extremitäten wiesen einen Rigor auf. Bei dem Versuch einer Willkür-Motorik traten häufig klonische Krämpfe auf.

Die nachfolgenden Verbindungen wurden jeweils an 2-3 der so vorbehandelten Tiere getestet. Bei intramuskulärer Gabe wurden nach etwa 5 bis 10 Minuten erste koordinierte Willkürbewegungen möglich, die in den folgenden 10 bis 30 Minuten von einer allmählichen, weitestgehenden Normalisierung der Motorik gefolgt waren. Die Tiere waren in der Lage, Nahrung aufzunehmen. Sie verhielten sich innerhalb ihrer Käfige regelgerecht, dies galt auch hinsichtlich Vigilanz und artspezifischen Verhaltens. Als Restsymptomatik wurde gelegentlich leichter Ruhetremor registriert.

Sobald die Wirkung der Verbindungen nachließ, verfielen die Tiere wieder in die oben beschriebene Parkinson-Symptomatik; eine erneute Applikation der Verbindungen führte wieder zur Besserung bzw. weitgehenden Aufhebung der klinischen Erscheinungen.

Nebenwirkungen traten in den bisher angewandten Dosierungen nicht in Erscheinung.

In der nachfolgenden Tabelle werden sowohl die Mindestdosen, die zur Aufhebung der Parkinsonsymptomatik führen, angegeben, als auch die Wirkungsdauer.

| | Dosis mg/kg i.m. | Dauer (Std.) |
|---|---|---|
| B | 0,05 | 2-5 |
| C | 0,35 | 2-4 |
| D | 0,03 | 2-3 |
| F | 0,2 | 2-3 |

Ferner sind die erfindungsgemäß hergestellten Verbindungen weitgehend untoxisch. So konnten z. B. bei der Untersuchung der Substanz C an Mäusen mit Dosen bis zu 500 mg/kg p.o. keine Todesfälle festgestellt werden.

Aufgrund ihrer pharmakologischen Eigenschaften eignen sich die erfindungsgemäß hergestellten Verbindungen der allgemeinen Formel I und deren physiologisch verträgliche Säureadditionssalze zur Behandlung von neuropsychiatrischen Erkrankungen, insbesondere aber zur Behandlung der Parkinson'schenerkrankung, der Schizophrenie und/oder zur Behandlung von Kreislauferkrankungen.

Zur pharmazeutischen Anwendung lassen sich die neuen Verbindungen und deren physiologisch verträgliche Säureadditionssalze, gegebenenfalls in Kombination mit anderen Wirkstoffen, in die üblichen galenischen Anwendungsformen wie Dragées, Tabletten, Pulver, Suppositorien, Suspensionen, Tropfen oder Ampullen einarbeiten. Die Einzeldosis beträgt hierbei 1 bis 4 $^{\times}$ täglich 0,01 bis 1,0 mg/kg Körpergewicht, vorzugsweise jedoch 0,1 bis 0,3 mg/kg Körpergewicht.

Die nachfolgenden Beispiele sollen die Erfindung näher erläutern:

Beispiel 1

2-Acetylamino-4-(1-n-propyl-1,2,3,6-tetrahydro-pyridin-4-yl)-thiazol

16,4 g (75 mMol) 2-Acetylamino-4-(pyridyl-4)thiazol werden in 150 ml absolutem Dimethylformamid gelöst und nach Zugabe von 66,4 g (0,54 Mol) n-Propylbromid 4 Stunden im Ölbad bei 140°C gerührt. Anschließend wird eingeengt, in 350 ml Ethanol gelöst und unter Kühlung portionsweise mit 4,97 g (0,131 Mol) Natriumborhydrid versetzt. Nach Rühren über Nacht wird mit Eis zersetzt, mit verdünnter Salzsäure - schwach angesäuert, dann mit wässrigem Ammoniak alkalisch gestellt und mit Chloroform extrahiert. Die Extrakte werden getrocknet, eingeengt und der Einengungsrückstand in Acetonitril zur Kristallisation gebracht.

Ausbeute: 18,5 g (93 % der Theorie),
Schmelzpunkt: 164-167°C
Ber.: C 58,84 H 7,22 N15,83 S12,08
Gef.: C 58,71 H 7,11 N15;73 S 11,97

Analog Beispiel 1 wurden hergestellt:

1a) 2-Acetylamino-4,(1-allyl-1,2,3,6-tetrahydro-pyridin-4-yl)thiazol

aus 2-Acetylamino-4-(pyridyl-4)thiazol und Allylbromid.
Ausbeute: 80 % der Theorie,
Schmelzpunkt: 165-170°C
Ber.: C 59,29 H 6,51 N 15,95 S 12,17
Gef.: C 59,11 H 6,47 N 15,75 S 12,05

1b) 2-Acetylamino-4-methyl-5-(1-n-propyl-1,2,3,6-tetrahydropyridin-5-yl)thiazol

aus 2-Acetylamino-4-methyl-5-(pyridyl-3)thiazol und n-Propylbromid.
Ausbeute: 33 % der Theorie,

Schmelzpunkt: 101-104°C

Ber.: C 60,18 H 7,57 N 15,05 S 11,48

Gef.: C 60,25 H 7,68 N14,95 S 11,35

1c) 2-Acetylamino-4-methyl-5-(1-n-propyl-1,2,3,6-tetrahydropyridin-4-yl)thiazol

aus 2-Acetylamino-4-methyl-5-(pyridyl-4)thiazol und n-Propylbromid.

Ausbeute: 76,9 % der Theorie,

Schmelzpunkt: 150-154°C

Ber.: C 60,18 H 7,57 N 15,05 S 11,48

Gef.: C 60,15 H 7,56 N 15,01 S 11,26

1d) 2-Acetylamino-4-(1-cinnamyl-1,2,3,6-tetrahydro-pyridin4-yl)thiazol

aus 2-Acetylamino-4-(pyridyl-4)thiazol und Cinnamylchlorid.

Ausbeute: 73,5 % der Theorie,

Schmelzpunkt: 156-160°C

Ber.: C 67,23 H 6,24 N 12,38 S 9,45

Gef.: C 67,16 H 6,19 N12,27 S 9,31

1e) 2-Acetylamino-4-(1-cinnamyl-1,2,3,6-tetrahydro-pyridin-5-yl)thiazol

aus 2-Acetylamino-4-(pyridyl-3)thiazol und Cinnamylchlorid.

Ausbeute: 67,5 % der Theorie,

Schmelzpunkt: 193-196°C

Ber.: C 67,23 H 6,24 N 12,38 S 9,45

Gef.: C 67,07 H 6,11 N 12,17 S 9,26

1f) 2-Acetylamino-4-[1-(3-methoxy-cinnamyl) -1,2,3,6-tetrahydro-pyridin-4-yl]thiazol-dihydrochlorid-hydrat

aus 2-Acetylamino-4-(pyridyl-4)thiazol und 3-Methoxy-cinnamylchlorid.

Ausbeute: 45 % der Theorie,

Schmelzpunkt: 157-159°C (Zers.)

Ber.: C 52,17 H 5,91 N 9,12 Cl 15,40

Gef.: C 51,99 H 5,92 N 9,17 Cl 15,20

1g) 2-Acetylamino-4-[1-(3-methoxy-cinnamyl)-1,2,3,6-tetrahydro-pyridin-5-yl]thiazol-dihydrochlorid

aus 2-Acetylamino-4-(pyridyl-3)thiazol und 3-Methoxy-cinnamylchlorid.

Ausbeute: 48 % der Theorie,

Schmelzpunkt: 266-268°C (Zers.)

Ber.: C 54,30 H 5,70 N 9,50 Cl 16,03

Gef.: C 54,34 H 5,80 N 9,38 Cl 16,27

1h) 2-Acetylamino-4-(1-n-hexyl-1,2,3,6-tetrahydro-pyridin4-yl)thiazol

aus 2-Acetylamino-4-(pyridyl-4)thiazol und n-Hexylbromid.

Ausbeute: 7g % der Theorie,

Schmelzpunkt: 125-127°C

Ber.: C 62,50 H 8,19 N 13,67 S 10,43

Gef.: C 62,35 H 8,06 N 13,51 S 10,27

1i) 2-Acetylamino-4-(1-n-propyl-1,2,3,6-tetrahydro-pyridin-5-yl)thiazol

aus 2-Acetylamino-4-(pyridyl-3)thiazol und n-Propylbromid.

Ausbeute: 65,7 % der Theorie,

Schmelzpunkt: 118-119°C

Ber.: C 58,84 H 7,22 N 15,83 S 12,08

Gef.: C 59,01 H 6.99 N 15,90 S 11,94

1j) 2-Acetylamino-4-(1-allyl-1,2,3,6-tetrahydro-pyridin-5-yl)thiazol

aus 2-Acetylamino-4-(pyridyl-3)thiazol und Allylbromid.

Ausbeute: 54,4 % der Theorie,

Schmelzpunkt: 151-153°C

| | | | |
|---|---|---|---|
| Ber.: C 59,29 | H 6,51 | N 15,95 | S 12,17 |
| Gef.: C 59,22 | H 6,30 | N 16,12 | S 11,95 |

1k) 2-Acetylamino-4-(1-ethyl-1,2,3,6-tetrahydro-pyridin-5-yl)thiazol x 1,5-Hydrochlorid

aus 2-Acetylamino-4-(pyridyl-3)thiazol und Diäthylsulfat (bei 140°C ohne Lösungsmittel), anschließende Reduktion mit Natriumborhydrid in Ethanol und Hydrochloridfällung in Aceton mit isopropanolischer Salzsäure.
Ausbeute: 25 % der Theorie,
Schmelzpunkt: 290°C (Zers.)

| | | | |
|---|---|---|---|
| Ber.: C 47,08 | H 6,09 | N 13,73 | S 10,47 |
| Gef.: C 47,38 | H 6,19 | N 13,62 | S 10,33 |

1l) 2-Acetylamino-4-[1-(4-chlor-benzyl)-1,2,3,6-tetrahydropyridin-5-yl]thiazol

aus 2-Acetylamino-4-(pyridyl-3)thiazol und 4-Chlor-benzylbromid.
Ausbeute: 44,7 % der Theorie,
Schmelzpunkt: 198-200°C

| | | | |
|---|---|---|---|
| Ber.: C 58,70 | H 5,22 | N 12,08 | S 9,22 |
| Gef.: C 58,96 | H 5,34 | N 12,18 | S 9,27 |

1m) 2-Acetylamino-4-(1-benzyl-1,2,3,6-tetrahydro-pyridin-5-yl)thiazol

aus 2-Acetylamino-4-(pyridyl-3)thiazol und Benzylbromid.
Ausbeute: 48 % der Theorie,
Schmelzpunkt: 126-132°C

| | | | |
|---|---|---|---|
| Ber.: C 65,15 | H 6,11 | N 13,41 | S 10,23 |
| Gef.: C 65,01 | H 6,13 | N 13,27 | S 10,16 |

## Beispiel 2

2-Amino-4-(1-n-propyl-1,2,3,6-tetrahydro-pyridin-4-yl)thiazol

4 g (15,1 mMol) 2-Acetylamino-4-(1-n-propyl-1,2,3,6-tetrahydro-pyridin-4-yl)thiazol werden in 40 ml Ethanol und 40 ml (80 mMol) 2N Natronlauge gelöst und 7 Stunden am Rückfluß erhitzt. Dann wird im Vakuum eingeengt, mit Wasser aufgenommen und das ausgefallene Produkt abgesaugt oder mit Chloroform extrahiert. Das so erhaltene Produkt wird anschließend aus Acetonitril umkristallisiert.
Ausbeute: 2,8 g (83 % der Theorie),
Schmelzpunkt: 121-122°C

| | | | |
|---|---|---|---|
| Ber.: C 59,16 | H 7,67 | N 18,81 | S 14,35 |
| Gef.: C 59,36 | H 7,79 | N 18,78 | S 14,39 |

Analog Beispiel 2 wurden hergestellt:

2a) 2-Amino-4-(1-allyl-1,2,3,6-tetrahydro-pyridin-4-yl)-thiazol

aus 2-Acetylamino-4-(1-allyl-1,2,3,6-tetrahydro-pyridin-4-yl)thiazol.
Ausbeute: 81,7 % der Theorie,
Schmelzpunkt: 134-136°C

| | | | |
|---|---|---|---|
| Ber.: C 59,70 | H 6,83 | N 18,99 | S 14,49 |
| Gef.: C 59,59 | H 6,96 | N 18,78 | S 14,26 |

2b) 2-Amino-4-methyl-5-(1-n-propyl-1,2,3,6-tetrahydro-pyridin-5-yl)thiazol

aus 2-Acetylamino-4-Methyl-5-(1-n-propyl-1,2,3,6-tetrahydropyridin-5-yl)thiazol.
Ausbeute: 47,5 % der Theorie,
Schmelzpunkt: 118-119°C

| | | | |
|---|---|---|---|
| Ber.: C 60,72 | H 8,07 | N 17,70 | S 13,51 |
| Gef.: C 60,78 | H 7,86 | N 17,62 | S 13,33 |

2c) 2-Amino-4-methyl-5-(1-n-propyl-1,2,3,6-tetrahydro-pyridin-4-yl)thiazol

aus 2-Acetylamino-4-methyl-5-(1-n-propyl-1,2,3,6-tetrahydropyridin-4-yl)thiazol
Ausbeute: 85,7 % der Theorie,
Schmelzpunkt: 157-160°C

| | | | |
|---|---|---|---|
| Ber.: C 60,72 | H 8,07 | N 17,70 | S 13,51 |
| Gef.: C 61,00 | H 7,93 | N 17,75 | S 13,78 |

2d) 2-Amino-4-(1-n-propyl-piperidin-4-yl)thiazol

aus 2-Acetylamino-4-(1-n-propyl-piperidin-4-yl)thiazol.
Ausbeute: 69 % der Theorie,
Schmelzpunkt: 131-133°C

| | | | |
|---|---|---|---|
| Ber.: C 58,63 | H 8,50 | N 18,65 | S 14,23 |
| Gef.: C 58,51 | H 8,45 | N 18,62 | S 14,28 |

2e) 2-Amino-4-(1-cinnamyl-1,2,3,6-tetrahydro-pyridin-4-yl)-thiazol-hydrat
aus 2-Acetylamino-4-(1-cinnamyl-1,2,3,6-tetrahydro-pyridin4-yl)thiazol.

Ausbeute: 62 % der Theorie,
Schmelzpunkt: 131-134°C

| | | | |
|---|---|---|---|
| Ber.: C 64,74 | H 6,71 | N 13,32 | S 10,16 |
| Gef.: C 64,77 | H 6,83 | N 13,24 | S 9,96 |

2f) 2-Amino-4-(1-cinnamyl-1,2,3,6-tetrahydro-pyridin-5-yl)-thiazol-dihydrochlorid

aus 2-Acetylamino-4-(1-cinnamyl-1,2,3,6-tetrahydro-pyridin-5-yl)thiazol.
Ausbeute: 30 % der Theorie,
Schmelzpunkt: 255-260°C (zers.)

| | | | |
|---|---|---|---|
| Ber.: C 55,13 | H 5,72 | N 11,34 | Cl 19,14 |
| Gef.: C 55,14 | H 5,94 | N 11,12 | Cl 19,06 |

2g) 2-Amino-4-[1-(3-methoxy-cinnamyl)-1,2,3,6-tetrahydropyridin-4-yl]thiazol-dihydrochlorid-semihydrat

aus 2-Acetylamino-4-[1-(3-methoxy-cinnamyl)-1,2,3,6-tetrahydro-pyridin-4-yl]thiazol-dihydrochlorid-hydrat.
Ausbeute: 65 % der Theorie,
Schmelzpunkt: 228-230°C (Zers.)

| | | | |
|---|---|---|---|
| Ber.: C 52,81 | H 5,91 | N 10,26 | Cl 17,32 |
| Gef.: C 52,99 | H 5,92 | N 10,32 | Cl 17,18 |

2h) 2-Amino-4-[1-(3-methoxy-cinnamyl)-1,2,3,6-tetrahydropyridin-5-yl]thiazol-dihydrochlorid-hydrat

aus 2-Acetylamino-4-[1-(3-methoxy-cinnamyl)-1,2,3,6-tetrahydro-pyridin-5-yl]thiazol-dihydrochlorid.
Ausbeute: 41 % der Theorie,
Schmelzpunkt: 201-203°C (Zers.)

| | | | |
|---|---|---|---|
| Ber.: C 51,67 | H 6,02 | N 10,04 | Cl 16,95 |
| Gef.: C 51,63 | H 6,13 | N 10,11 | Cl 16,72 |

2i) 2-Amino-4-(1-n-hexyl-1,2,3,6-tetrahydro-pyridin-4-yl)-thiazol-dihydrochlorid-semihydrat

aus 2-Acetylamino-4-(1-n-hexyl-1,2,3,6-tetrahydro-pyridin-4-yl)thiazol.
Ausbeute: 85 % der Theorie,
Schmelzpunkt: 175-176°C (Zers.)

| | | | |
|---|---|---|---|
| Ber.: C 48,41 | H 7,54 | N 12,10 | Cl 20,41 |
| Gef.: C 48,42 | H 7,56 | N 12,24 | Cl 20,22 |

2j) 2-Amino-4-(1-n-propyl-1,2,3,6-tetrahydro-pyridin-5-yl)-thiazol

aus 2-Acetylamino-4-(1-n-propyl-1,2,3,6-tetrahydro-pyridin-5-yl)thiazol.
Ausbeute: 72,4 % der Theorie,
Schmelzpunkt: 124-126°C

| | | | |
|---|---|---|---|
| Ber.: C 59,16 | H 7,67 | N 18,81 | S 14,35 |
| Gef.: C 59,30 | H 7,83 | N 18,74 | S 14,56 |

2k) 2-Amino-4-(1-allyl-1,2,3,6-tetrahydro-pyridin-5-yl)thiazol

aus 2-Acetylamino-4-(1-allyl-1,2,3,6-tetrahydro-pyridin-5-yl)thiazol.
Ausbeute: 54,4 % der Theorie,
Schmelzpunkt: 132-133°C

| | | | |
|---|---|---|---|
| Ber.: C 59,70 | H 6,83 | N 18,99 | S 14,49 |
| Gef.: C 59,80 | H 6,93 | N 19,11 | S 14,22 |

2l) 2-Amino-4-(1-ethyl-1,2,3,6-tetrahydro-pyridin-5-yl)-thiazol

aus 2-Acetylamino-4-(1-ethyl-1,2,3,6-tetrahydro-pyridin-5-yl)thiazol × 1,5 Hydrochlorid.
Ausbeute: 62,8 % der Theorie,
Schmelzpunkt: 142-144°C

| | | | |
|---|---|---|---|
| Ber.: C 57,38 | H 7,22 | N 20,08 | S 15,32 |
| Gef.: C 57,20 | H 7,28 | N 19,99 | S 15,24 |

2m) 2-Amino-4-[1-(4-chlor-benzyl)-1,2,3,6-tetrahydro-pyridin-5-yl]-thiazol

aus 2-Acetylamino-4-[1-(4-Chlor-benzyl)-1,2,3,6-tetrahydropyridin-5-yl]thiazol.
Ausbeute: 88,4 % der Theorie,
Schmelzpunkt: 162-164°C

| | | | |
|---|---|---|---|
| Ber.: C 58,91 | H 5,27 | N 13,74 | S 10,48 |
| Gef.: C 58,90 | H 5,13 | N 13,92 | S 10,57 |

2n) 2-Amino-4-(1-n-propyl-piperidin-3-yl)thiazol-dihydrochlorid

aus 2-Acetylamino-4-(1-n-propyl-piperidin-3-yl)thiazol.
Ausbeute: 52 % der Theorie,
Schmelzpunkt: 247-249°C (Zers.)

| | | | |
|---|---|---|---|
| Ber.: C 44,29 | H 7,10 | N 14,09 | S 10,75 |
| Gef.: C 44,22 | H 6,94 | N 14,13 | S 10,94 |

## Beispiel 3

2-Acetylamino-4-(1-n-propyl-piperidin-4-yl)thiazol

18,4 g (69,3 mMol) 2-Acetylamino-4-(1-n-propyl-1,2,3,6-tetrahydro-pyridin-4-yl)thiazol werden in 1000 ml Methanol gelöst und nach Zugabe von 20 g Palladium (10%ig auf Kohle) bei 40°C unter einem Druck von 5 bar Wasserstoff hydriert. Anschließend wird vom Katalysator abfiltriert und eingeengt. Der Rückstand wird aus wenig Acetonitril umkristallisiert.
Ausbeute: 14,4 g (77,8 % der Theorie),
Schmelzpunkt: 135-137°C

| | | | |
|---|---|---|---|
| Ber.: C 58,40 | H 7,92 | N 15,71 | S 11,99 |
| Gef.: C 58,11 | H 7,91 | N 15,56 | S 11,76 |

Analog Beispiel 3 wurden hergestellt:

3a) 2-Amino-4-(1-n-propyl-piperidin-4-yl)thiazol

aus 2-Amino-4-(1-n-propyl-1,2,3,6-tetrahydro-pyridin-4-yl)thiazol.
Ausbeute: 61 % der Theorie,
Schmelzpunkt: 131-133°C

| | | | |
|---|---|---|---|
| Ber.: C 58,63 | H 8,50 | N 18,65 | S 14,23 |
| Gef.: C 58,55 | H 8,49 | N 18,53 | S 14,07 |

3b) 2-Amino-4-methyl-5-(1-n-propyl-piperidin-4-yl)thiazol

aus 2-Amino-4-methyl 5-(1-n-propyl-1,2,3,6-tetrahydro-pyridin-4-yl)thiazol.
Ausbeute: 61,6 % der Theorie,

Schmelzpunkt: 175-178°C
Ber.: C 60,21 H 8,84 N 17,55 S 13,39
Gef.: C 60,40 H 8,64 N 17,87 S 13,20

3c) 2-Amino-4-methyl-5-(1-n-propyl-piperidin-3-yl)thiazol

aus 2-Amino-4-methyl 5-(1-n-propyl-1,2,3,6-tetrahydro-pyridin-5-yl)thiazol.
Ausbeute: 69,4 % der Theorie,
Schmelzpunkt: 119-121°C
Ber.: C 60,21 H 8,84 N 17,55 S 13,39
Gef.: C 60,25 H 8,93 N 17,34 S 13,24

3d) 2-Acetylamino-4-(1-n-propyl-piperidin-3-yl)thiazol

aus 2-Acetylamino-4-(1-n-propyl-1,2,3,6-tetrahydro-pyridin-5-yl)thiazol.
Ausbeute: 82 % der Theorie,
Schmelzpunkt: 125-127°C
Ber.: C 58,40 H 7,92 N 15,71 S 11,99
Gef.: C 58,36 H 7,79 N 15,46 S 11,76

3e) 2-Acetylamino-4-(1-benzyl-piperidin-3-yl)thiazol-hydrochlorid

aus 2-Acetylamino-4-(1-benzyl-1,2,3,6-tetrahydro-pyridin-5-yl)thiazol und nachträgliche Fällung als Hydrochlorid in Methylenchlorid/Ether.
Ausbeute: 45 % der Theorie,
Schmelzpunkt: 265°C
Ber.: C 58,02 H 6,30 N 11,94 S 9,11
Gef.: C 57,83 H 6,20 N 11,88 S 8,94

3f) 2-Acetylamino-4-(1-n-propyl-piperidin-3-yl)thiazol-dihydrochlorid

aus 2-Acetylamino-4-(1-allyl-1,2,3,6-tetrahydro-pyridin-5-yl)thiazol und nachträgliche Fällung als Dihydrochlorid in Aceton mit isopropanolischer Salzsäure.
Ausbeute: 35 % der Theorie,
Schmelzpunkt: 270-275°C
Ber.: C 45,88 H 6,81 N 12,35 S 9,42
Gef.: C 45,62 H 6,93 N 12,41 S 9,63

3g) 2-Amino-4-(1-n-propyl-piperidin-3-yl)thiazol-dihydrochlorid

aus 2-Amino-4-(1-n-propyl-1,2,3,6-tetrahydro-pyridin-5-yl)thiazol und nachträgliche Fällung als Dihydrochlorid.
Ausbeute: 64 % der Theorie,
Schmelzpunkt: 246-249°C (Zers.)
Ber.: C 44,29 H 7,10 N 14,09 S 10,53
Gef.: C 44,11 H 7,02 N 14,03 S 10,69

Beispiel 4

2-Acetylamino-4-(1,2,3,6-tetrahydro-pyridin-5-yl)thiazol-dihydrobromid

A) 2-Acetylamino-4-(1-benzyloxycarbonyl-1,2,3,6-tetrahydropyridin-5-yl)thiazol

19,0 g (60,7 mMol) 2-Acetylamino-4-(1-benzyl -1,2,3,6-tetrahydro-pyridin-5-yl)thiazol werden in 250 ml Chloroform gelöst und bei 0-5°C mit 42,5 g (0,25 Mol) Chlorameisensäure-benzylester tropfenweise versetzt. Anschließend rührt man über Nacht bei Raumtemperatur, engt dann ein und reinigt den Einengungsrückstand durch Säulenchromatographie an Kieselgel (Fließmittel: Toluol/Essigsäure-ethylester = 2:1).
Ausbeute: 9,25 g (42,6 % der Theorie),
Schmelzpunkt: 181-183°C
Ber.: C 60,49 H 5,36 N 11,76 S 8,97
Gef.: C 60,31 H 5,27 N 11,68 S 8,76

B) 2-Acetylamino-4-(1,2,3,6-tetrahydro-pyridin-5-yl)thiazoldihydrobromid

3,57 g (10 mMol) 2-Acetylamino-4-(1-benzyloxycarbonyl-1,2,3,6-tetrahydro-pyridin-5-yl)thiazol werden mit 10 ml 33%iger Bromwasserstoffsäure in Eisessig versetzt und 8 Stunden bei Raumtemperatur gerührt. Dann wird mit Diethylether versetzt und vom ausgefallenen Produkt abgesaugt.
Ausbeute: 3,57 g (93 % der Theorie),
Schmelzpunkt: 202°C (Zers.)

| | | | |
|---|---|---|---|
| Ber.: C 31,18 | H 3,93 | N 10,91 | S 8,33 |
| Gef.: C 31,30 | H 4,02 | N 11,10 | S 8,45 |

Durch Versetzen einer wässrigen Lösung des so gewonnenen Dihydrobromids mit konzentriertem Ammoniak fällt das 2-Acetylamino-4-(1,2,3,6-tetrahydro-pyridin-5-yl)thiazol als freie Base aus.
Ausbeute: 75 % der Theorie,
Schmelzpunkt: 233-236°C

Analog Beispiel 4 wurden hergestellt:

4aA) 2-Acetylamino-4-(1-benzyloxycarbonyl-1,2,3,6-tetrahydropyridin-4-yl)thiazol

aus 2-Acetylamino-4-(1-benzyl-1,2,3,6-tetrahydro-pyridin-4-yl)thiazol und Chlorameisensäure-benzylester.
Ausbeute: 38 % der Theorie,
Schmelzpunkt: 186-188°C

| | | | |
|---|---|---|---|
| Ber.: C 60,49 | H 5,36 | N 11,76 | S 8,97 |
| Gef.: C 60,44 | H 5,38 | N 11,79 | S 8,71 |

4aB) 2-Acetylamino-4-(1,2,3,6-tetrahydro-pyridin-4-yl)thiazol-dihydrobromid

aus 2-Acetylamino-4-(1-benzyloxycarbonyl-1,2,3,6-tetrahydropyridin-4-yl)thiazol.
Ausbeute: 91 % der Theorie,
Schmelzpunkt: 218-222°C

| | | | |
|---|---|---|---|
| Ber.: C 31,18 | H 3,93 | N 10,91 | S 8,33 |
| Gef.: C 31,03 | H 4,09 | N 10,79 | S 8,35 |

Schmelzpunkt der freien Base: 234-237°C (Zers.)

Beispiel 5

2-Amino-4-(1,2,3,6-tetrahydro-pyridin-5-yl)thiazol-dihydrochlorid

3,0 g (7,8 mMol) 2-Acetylamino-4-(1,2,3,6-tetrahydro-pyridin-5-yl)thiazol-dihydrobromid werden in 50 ml halbkonzentrierter Salzsäure 4 Stunden auf 100°C erhitzt. Anschließend wird eingeengt, mit Toluol versetzt, erneut eingeengt und mit Aceton zur Kristallisation gebracht Zur Reinigung wird das Kristallisat in Ethanol aufgekocht und nach dem Abkühlen abgesaugt.
Ausbeute: 1,8 g (91 % der Theorie),
Schmelzpunkt: 295-297°C (Zers.)

| | | | |
|---|---|---|---|
| Ber.: C 37,80 | H 5,16 | N 16,53 | S 12,62 |
| Gef.: C 37,50 | H 5,02 | N 16,31 | S 12,47 |

Beispiel 6

2-Amino-4-(1-n-propyl-piperidin-4-yl)thiazol

Zu 0,84 g (8,3 mMol) Diisopropylamin in 8 ml absolutem Glykoldimethylether tropft man unter Stickstoff und Kühlung (0°C) 5,2 ml (8,3 mMol) einer 1,6 molaren Lösung von Butyllithium in Hexan. Anschließend gibt man zuerst 1,4 g (8,3 mMol) 4-Acetyl-1-n-propyl-piperidin, dann eine filtrierte Lösung von 0,42 ml Triethylamin und 1,66 ml Trimethylchlorsilan in 4 ml Glykoldimethylether zu. Nach 30 Minuten Rühren wird mit Natriumhydrogencarbonatlösung zersetzt und mit Ether extrahiert. Die Extrakte werden getrocknet und eingeengt und der Einengungsrückstand, nach Aufnahme in 10 ml Methylenchlorid, tropfenweise mit 1,32 g

(8 mMol) Brom versetzt. Danach fügt man 0,6 g (8 mMol) Thioharnstoff und 1,0 g Kaliumacetat in 20 ml Ethanol zu und kocht 3 Stunden am Rückfluß. Anschließend wird eingeengt, mit verdünnter Natronlauge versetzt und mit Methylenchlorid extrahiert. Die Extrakte werden getrocknet und eingeengt und der Einengungsrückstand an Kieselgel säulenchromatographisch gereinigt (Fließmittel: Chloroform/Methanol/methanolischer Ammoniak = 6:1:0,1).
Ausbeute: 640 mg (34 % der Theorie),
Schmelzpunkt: 130-132°C

| | | | |
|---|---|---|---|
| Ber.: C 58,63 | H 8,50 | N 18,65 | S 14,23 |
| Gef.: C 58,59 | H 8,49 | N 18,47 | S 14,08 |

Beispiel 7

2-Acetylamino-4-(1-n-propyl-1,2,3,6-tetrahydro-pyridin-4-yl)-thiazol

1 g (2,6 mMol) 2-Acetylamino-4-(1,2,3,6-tetrahydro-pyridin-4-yl)thiazol-dihydrobromid werden in 25 ml Chloroform suspendiert und nach Zugabe von 2,0 ml (11,6 mMol) Diisopropylethylamin und 1 ml (10,3 mMol) n-Propyljodid 5 Stunden am Rückfluß gekocht. Dann wird eingeengt und der Einengungsrückstand an Kieselgel säulenchromatographisch gereinigt (fließmittel: Essigsäure-ethylester/Methanol = 10:1).
Ausbeute: 270 mg (3g % der Theorie),
Schmelzpunkt: 164-166°C

| | | | |
|---|---|---|---|
| Ber.: C 58,84 | H 7,22 | N 15,83 | S 12,08 |
| Gef.: C 58,76 | H 7,11 | N 15,67 | S 11,99 |

Beispiel 8

2-Acetylamino-4-(1-n-propyl-1,2,3,6-tetrahydro-pyridin-4-yl)-thiazol

Eine Lösung von 225 mg (1 mMol) 2-Acetylamino-4-(1,2,3,6-tetrahydro-pyridin-4-yl)thiazol in 2,5 ml Dimethylformamid wird mit 70 mg (1,2 mMol) Propionaldehyd versetzt und 1 Stunde auf 40-50°C erwärmt. Dann gibt man 50 mg (1,3 mMol) Natriumborhydrid zu und erwärmt weitere 30 Minuten auf 40-50°C. Anschließend gibt man auf 2N Salzsäure, rührt 10 Minuten bei Raumtemperatur, stellt dann mit konzentriertem Ammoniak alkalisch und extrahiert mit Chloroform. Die Extrakte werden getrocknet, eingeengt und der Einengungsrückstand an Kieselgel säulenchromatographisch gereinigt (Fließmittel: Essigsäure-ethylester/Methanol = 10:1).
Ausbeute: 70 mg (26 % der Theorie),
Schmelzpunkt: 164-166°C

| | | | |
|---|---|---|---|
| Ber.: C 58,84 | H 7,22 | N 15,83 | S 12,08 |
| Gef.: C 58,98 | H 7,32 | N 15,79 | S 12,01 |

Beispiel 9

2-[N-(3-Chlorbenzyl)amino]-4-(1-allyl-1,2,3,6-tetrahydro-pyridin-4-yl)thiazol

440 mg (2 mMol) 2-Amino-4-(1-allyl-1,2,3,6-tetrahydro-pyridin-4-yl)thiazol werden in 25 ml Toluol gelöst und nach Zugabe von 700 mg (5 mMol) 3-Chlor-benzaldehyd und 50 mg p-Toluolsulfonsäure 4 Stunden am Wasserabscheider gekocht. Nach Abkühlen des Reaktionsgemisches gibt man 50 ml Ethanol und dann 500 mg (13,0 mMol) Natriumborhydrid portionsweise unter leichter Kühlung zu. Nach Rühren über Nacht wird mit 2 N Salzsäure angesäuert und mit Essigsäure-ethylester extrahiert. Anschließend wird die wässrige Phase ammoniakalisch gestellt und mit Methylenchlorid extrahiert. Die Extrakte werden getrocknet und eingeengt. Der Einengungsrückstand wird aus Essigsäure-ethylester umkristallisiert.
Ausbeute: 480 mg (69,5 % der Theorie),
Schmelzpunkt: 132-133°C

| | | | |
|---|---|---|---|
| Ber.: C 62,51 | H 5,83 | N 12,15 | S Cl 10,25 |
| Gef.: C 62,43 | H 5,75 | N 12,07 | S Cl 10,17 |

Beispiel 10

2-[N-(4-Chlorbenzoyl)amino]-4-(1-allyl-1,2,3,6-tetrahydro-pyridin-4-yl)thiazol

550 mg (2,5 mMol) 2-Amino-4-(1-allyl-1,2,3,6-tetrahydro-pyri din-4-yl)thiazol werden in 50 ml Chloroform gelöst und nach Zugabe von 0,5 ml (2,9 mMol) Diisopropyl-ethylamin tropfenweise mit 450 mg (2,6 mMol) 4-Chlor-benzoylchlorid versetzt. Nach 3-stündigem Rühren bei Raumtemperatur wird eingeengt und der Einengungsrückstand an Kieselgel säulenchromatographisch gereinigt (Fließmittel: Chloroform/Methanol = 20:1). Anschließend wird aus Acetonitril umkristallisiert.
Ausbeute: 750 mg (83 % der Theorie),
Schmelzpunkt: 126-128°C

| | | | |
|---|---|---|---|
| Ber.: C 60,08 | H 5,04 | N 11,68 | Cl 9,85 |
| Gef.: C 60,01 | H 5,10 | N 11,57 | Cl 9,76 |

Beispiel 11

2-[N-(4-Chlorbenzyl)amino]-4-(1-allyl-1,2,3,6-tetrahydro-pyridin-4-yl)thiazol

Zur Suspension von 340 mg (8,9 mMol) Lithiumaluminiumhydrid in 100 ml absolutem Tetrahydrofuran gibt man 460 mg (1,28 mMol) 2-[N-(4-Chlorbenzoyl)amino]-4-(1-allyl-1,2,3,6-tetrahydro-pyridin-4-yl)thiazol und kocht dann 2 Stunden am Rückfluß. Anschließend wird mit 2 N Natronlauge zersetzt, vom gebildeten Natriumaluminat abgesaugt und das Filtrat eingeengt. Der Einengungsrückstand wird aus Essigsäure-ethylester umkristallisiert.
Ausbeute: 150 mg (33,8 % der Theorie),
Schmelzpunkt: 144-146°C

| | | | |
|---|---|---|---|
| Ber.: C 62,51 | H 5,83 | N 12,15 | Cl 10,25 |
| Gef.: C 62,43 | H 5,79 | N 12,01 | Cl 10,13 |

Beispiel 12

2-Acetylamino-4-(1-n-butyl-1,2,3,6-tetrahydro-pyridin-5-yl)-thiazol-hydrochlorid

1 g (2,6 mMol) 2-Acetylamino-4-(1,2,3,6-tetrahydro-pyridin-5-yl)thiazol-dihydrobromid werden in 30 ml Ethanol gelöst und nach Zugabe von 1,0 ml (5,3 mMol) n-Butylbromid und 1,5 g (10,8 mMol) Kaliumcarbonat 4 Stunden am Rückfluß gekocht. Nach dem Einengen wird mit Wasser versetzt und mit Chloroform extrahiert. Die Chloroformextrakte werden getrocknet und eingeengt und der Einengungsrückstand an Kieselgel säulenchromatographisch gereinigt (Fließmittel: Essigsäure-ethylester/Methanol = 10:1). Anschließend wird in Aceton das Hydrochlorid gefällt.
Ausbeute: 130 mg (16 % der Theorie),
Schmelzpunkt: 292-294°C
Ber.: m/e = 279
Gef.: m/e = 279

Beispiel I

Dragéekern mit 5 mg 2-Amino-4-(1-n-propyl-1,2,3,6-tetrahydropyridin-4-yl)thiazol

Zusammensetzung:
1 Dragéekern enthält:

| | |
|---|---|
| Wirksubstanz | 5,0 mg |
| Milchzucker | 33,5 mg |
| Maisstärke | 10,0 mg |
| Gelatine | 1,0 mg |
| Magnesiumstearat | 0,5 mg |
| | 50,0 mg |

Herstellungsverfahren

Die Mischung der Wirksubstanz mit Milchzucker und Maisstärke wird mit einer 10%igen wässrigen Gelatinelösung durch ein Sieb von 1 mm Maschenweite granuliert, bei 40°C getrocknet und nochmals durch obiges Sieb gerieben. Das so erhaltene Granulat wird mit Magnesiumstearat gemischt und zu Dragéekernen verpreßt. Die Herstellung muß in abgedunkelten Räumen vorgenommen werden.

Kerngewicht: 50 mg
Stempel: 5 mm, gewölbt

Die so erhaltenen Dragéekerne werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht: 100 mg

Beispiel II

Tropfen mit 5 mg 2-Amino-4-(1-n-propyl-1,2,3,6-tetrahydropyridin-4-yl)thiazol-dihydrochlorid

Zusammensetzung: 100 ml Tropfsubstanz enthalten:

| | |
|---|---|
| p-Hydroxybenzoesäure-methylester | 0,035 g |
| p-Hydroxybenzoesäure-n-propylester | 0,015 g |
| Anisöl | 0,05 g |
| Menthol | 0,06 g |
| Ethanol rein | 10,0 g |
| Wirksubstanz | 0,5 g |
| Zitronensäure | 0,7 g |
| Natriumphosphat sek. × 2 $H_2O$ | 0,3 g |
| Natriumcyclamat | 1,0 g |
| Glycerin | 15,0 g |
| Dest. Wasser | ad 100,0 ml |

Herstellungsverfahren:

Die p-Hydroxybenzoesäureester, Anisöl sowie Menthol werden in Ethanol gelöst (Lösung I).
Die Puffersubstanzen, die Wirksubstanz und Natriumcyclamat werden in dest. Wasser gelöst und Glycerin zugefügt (Lösung II). Lösung I wird in Lösung II eingerührt und die Mischung mit dest. Wasser auf das gegebene Volumen aufgefüllt. Die fertige Tropflösung wird durch ein geeignetes Filter filtriert. Die Herstellung und Abfüllung der Tropflösung muß unter Lichtschutz und unter Schutzbegasung erfolgen.

Beispiel III

Suppositorien mit 10 mg 2-Amino-4-(1-n-propyl-1,2,3,6-tetrahydro-pyridin-4-yl)thiazol

| 1 Zäpfchen enthält: | |
|---|---|
| Wirksubstanz | 10,0 mg |
| Zäpfchenmassen (z.B. Witepsol W 45) | 1 690,0 mg |
| | 1 700.0 mg |

Herstellungsverfahren:

Die feingepulverte Substanz wird mit Hilfe eines Eintauch homogenisators in die geschmolzene und auf 40°C abgekühlte Zäpfchenmasse eingerührt. Die Masse wird bei 35°C in leicht vorgekühlte Formen ausgegossen.

Zäpfchengewicht: 1,7 g

Beispiel IV

Ampullen mit 5 mg 2-Amino-4-(1-n-propyl-1,2,3,6-tetrahydropyridin-4-yl)thiazol-dihydrochlorid1 Ampulle enthält:

Wirksubstanz 5,0 mg
Zitronensäure 7,0 mg
Natriumphosphat sek. × 2 $H_2O$ 3,0 mg
Natriumpyrosulfit 1,0 mg
Dest. Wasser ad 1,0 ml

Herstellungsverfahren:

In ausgekochtem und unter $CO_2$-Begasung abgekühltem Wasser werden nacheinander die Puffersubstanzen, die Wirksubstanz sowie Natriumpyrosulfit gelöst. Man füllt mit abgekochtem Wasser auf das gegebene Volumen auf und filtriert pyrogenfrei.

Abfüllung: in braune Ampullen unter Schutzbegasung

Sterilisation: 20 Minuten bei 120°C

Die Herstellung und Abfüllung der Ampullenlösung muß in abgedunkelten Räumen vorgenommen werden.

Beispiel V

Dragées mit 1 mg 2-Amino-4-(1-n-propyl-1,2,3,6-tetrahydropyridin-4-yl)thiazol

| 1 Dragéekern enthält: | |
|---|---|
| Wirksubstanz | 1,0 mg |
| Milchzucker | 35,5 mg |
| Maisstärke | 12,0 mg |
| Gelatine | 1,0 mg |
| Magnesiumstearat | 0,5 mg |
| | 50,0 mg |

Herstellungsverfahren:

Analog Beispiel I. Kerngewicht: 50 mg
Stempel: 5 mm, gewölbt
Dragéegewicht: 100 mg

Als Wirkstoffe können in die pharmazeutischen Anwendungsbeispiele I bis V selbstverständlich an Stelle der genannten Verbindung alle übrigen Verbindungen der allgemeinen Formel I eingearbeitet werden.

**Ansprüche**

1. Piperidine der allgemeinen Formel

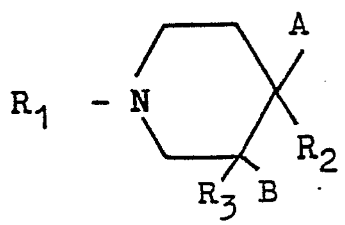

,(I)

in der
A und B jeweils ein Wasserstoffatom oder zusammen eine weitere Kohlenstoff-Kohlenstoff-Bindung,
$R_1$ ein Wasserstoffatom, eine gegebenenfalls durch eine Phenylgruppe substituierte Alkylgruppe mit 1 bis 3 Kohlenstoffatomen, eine Alkylgruppe mit 4 bis 6 Kohlenstoffatomen, eine gegebenenfalls durch eine Phenylgruppe substituierte Alkenyl-oder Alkinylgruppe mit jeweils 3 bis 5 Kohlenstoffatomen,

einer der Reste $R_2$ oder $R_3$ eine Gruppe der Formel

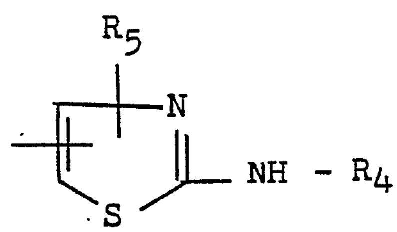

und der andere der Reste $R_2$ oder $R_3$ ein Wasserstoffatom, wobei $R_4$ ein Wasserstoffatom, eine gegebenenfalls durch eine Phenylgruppe substituierte Alkylgrupp mit 1 bis 3 Kohlenstoffatomen oder eine gegebenenfalls durch eine Phe nylgruppe substituierte Alkanoylgruppe mit 1 bis 4 Kohlenstoffatomen, wobei die vorstehend bei der Definition der Reste $R_1$ und $R_4$ erwähnten Phenylkerne jeweils durch ein Halogenatom, eine Methyl-oder Methoxygruppe substituiert sein können, und
$R_5$ ein Wasserstoffatom oder eine Alkylgruppe mit 1 bis 3 Kohlenstoffatomen darstellen, deren Enantiomere und deren Säureadditionssalze.

2. Piperidine der allgemeinen Formel I gemäß Anspruch 1, in der
A, B und $R_1$ wie im Anspruch 1 definiert sind,
$R_2$ eine Gruppe der Formel

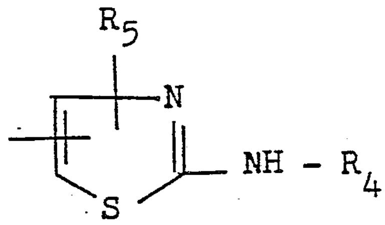

und $R_3$ ein Wasserstoffatom oder $R_3$ eine Gruppe der Formel

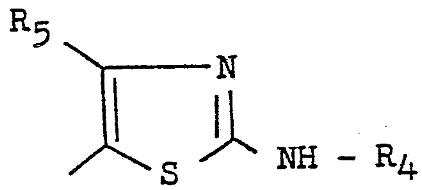

oder auch,
wenn $R_1$ ein Wasserstoffatom, eine durch eine Phenylgruppe substituierte Alkenyl-oder Alkinylgruppe mit jeweils 3 bis 5 Kohlenstoffatomen oder auch eine Alkylgruppe mit 1 bis 6 Kohlenstoffatomen, falls $R_4$ eine durch eine Phenylgruppe substituierte Alkylgruppe mit 1 bis 3 Kohlenstoffatomen oder eine durch eine Phenylgruppe substituierte Alkanoylgruppe mit 1 bis 4 Kohlenstoffatomen darstellt,
eine Gruppe der Formel

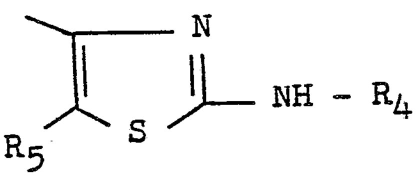

und $R_2$ ein Wasserstoffatom, wobei $R_4$ ein Wasserstoffatom, eine gegebenenfalls durch eine Phenylgruppe substituierte Alkylgruppe mit 1 bis 3 Kohlenstoffatomen oder eine gegebenenfalls durch eine Phenylgruppe substituierte Alkanoylgruppe mit 1 bis 4 Kohlenstoffatomen und
$R_5$ ein Wasserstoffatom oder eine Alkylgruppe mit 1 bis 3 Kohlenstoffatomen darstellen, wobei zusätzlich die bei der Definition der Reste $R_1$ und $R_4$ vorstehend erwähnten Phenylkerne jeweils durch ein Halogenatom, eine Methyl-oder Methoxygruppe substituiert sein können, deren Enantiomere und deren Säureadditionssalze.

3. Piperidine der allgemeinen Formel I gemäß Anspruch 1, in der A und B wie im Anspruch 2 definiert sind,
$R_1$ ein Wasserstoffatom, eine Alkylgruppe mit 1 bis 5 Kohlenstoffatomen, eine Allyl-, Cinnamyl-, Methoxycinnamyl-, Benzyl-oder Chlorbenzylgruppe,
$R_2$ eine Gruppe der Formel

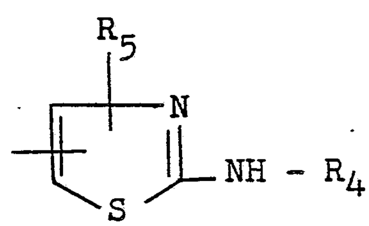

und $R_3$ ein Wasserstoffatom oder auch,
wenn $R_1$ ein Wasserstoffatom, eine Cinnamyl-oder Methoxycinnamylgruppe darstellt,

$R_3$ eine Gruppe der Formel

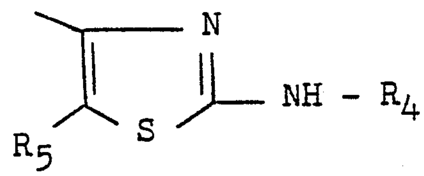

und $R_2$ ein Wasserstoffatom, wobei $R_4$ ein Wasserstoffatom, eine Chlorbenzylgruppe oder eine Alkanoylgruppe mit 2 oder 3 Kohlenstoffatomen und $R_5$ ein Wasserstoffatom oder eine Methylgruppe darstellen, deren Enantiomere und deren Säureadditionssalze.

4. 2-Amino-4-(1-n-propyl-piperidin-4-yl)-thiazol und dessen Säureadditionssalze.

5. 2-Amino-4-(1-n-propyl-1,2,3,6-tetrahydro-pyridin-4-yl)-thiazol und dessen Säureadditionssalze.

6. 2-Amino-4-(1-allyl-1,2,3,6-tetrahydro-pyridin-4-yl)thiazol und dessen Säureadditionssalze.

7. Physiologisch verträgliche Säureadditionssalze der Verbindungen gemäß den Ansprüchen 1 bis 6 mit anorganischen oder organischen Säuren.

8. Arzneimittel, enthaltend als Wirkstoff eine Verbindung gemäß den Ansprüchen 1 bis 6 oder ein physiologisch verträgliches Säureadditionssalz gemäß Anspruch 7 neben gegebenenfalls einem oder mehreren inerten Trägerstoffen und/oder Verdünnungsmitteln.

9. Verwendung einer Verbindung gemäß den Ansprüchen 1 bis 6 oder eines physiologisch verträglichen Säureadditionssalzes gemäß Anspruch 7 zur Herstellung eines Arzneimittels, welches zur Behandlung von zentralnervösen neuropsychiatrischen Erkrankungen und/oder zur Behandlung von Kreislauferkrankungen geeignet ist.

10. Verwendung einer Verbindung gemäß den Ansprüchen 1 bis 6 oder eines physiologisch verträglichen Säureadditionssalzes gemäß Anspruch 7 zur Herstellung eines Arzneimittels, welches zur Behandlung der Parkinson'schen-Erkrankung geeignet ist.

11. Verfahren zur Herstellung eines Arzneimittels, dadurch gekennzeichnet, daß auf nichtchemischem Wege eine Verbindung gemäß den Ansprüchen 1 bis 6 oder ein physiologisch verträgliches Säureadditionssalz gemäß Anspruch 7 in einen oder mehrere inerte Trägerstoffe und/oder Verdünnungmittel eingearbeitet wird.

12. Verfahren zur Herstellung der Piperidine gemäß den Ansprüchen 1 bis 7, dadurch gekennzeichnet, daß

a.) zur Herstellung von Verbindungen der allgemeinen Formel I, in der A und B zusammen eine weitere Kohlenstoff-Kohlenstoff-bindung darstellen, eine Pyridiniumverbindung der allgemeinen Formel

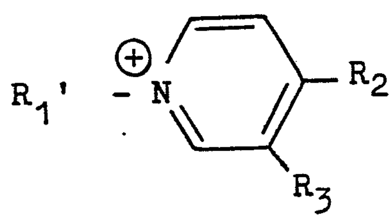

,(II)

in der

$R_2$ und $R_3$ wie in den Ansprüchen 1 bis 6 definiert

$R_1'$ mit Ausnahme des Wasserstoffatoms die für $R_1$ ein gangs erwähnten Bedeutungen besitzt, mit einem komplexen Metallhydrid reduziert wird oder

b) zur Herstellung von Verbindungen der allgemeinen Formel I, in der A und B jeweils Wasserstoffatome darstellen, eine Verbindung der allgemeinen Formel

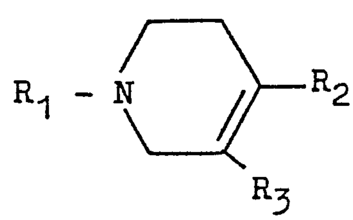

,(III)

in der
$R_1'$ $R_2$ und $R_3$ wie in den Ansprüchen 1 bis 6 definiert sind, katalytisch hydriert wird oder

c) zur Herstellung von Verbindungen der allgemeinen Formel I, in der $R_1$ und/oder $R_4$ ein Wasserstoffatom darstellen, ein oder zwei Schutzreste von einer Verbindung der allgemeinen Formel

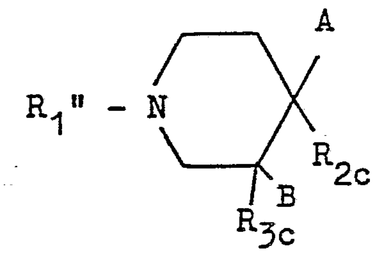
,(IV)

in der
A und B wie in den Ansprüchen 1 bis 6 definiert sind, $R_1''$ einen hydrolytisch abspaltbaren Rest oder die für $R_1$ in den Ansprüchen 1 bis 6 erwähnten Bedeutungen besitzt, einer der Reste $R_{2c}$ oder $R_{3c}$ eine Gruppe der Formel

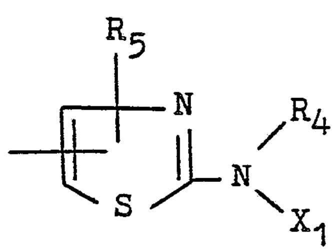
,

wobei $R_5$ wie in den Ansprüchen 1 bis 6 definiert ist,
$R_4'$ ein Wasserstoffatom oder einen der für $R_4$ in den Ansprüchen 1 bis 6 erwähnten Alkyl-oder gegebenenfalls substituierten Phenylalkylreste und
$X_1$ ein Wasserstoffatom, einen hydrolytisch abspaltbaren Rest oder eine gegebenenfalls durch eine Phenylgruppe substituierte Alkanoylgruppe mit 1 bis 4 Kohlenstoffatomen bedeuten, wobei jedoch mindestens einer der Reste $R_1''$ oder $X_1$ einen hydrolytisch abspaltbaren Rest darstellen muß,
und der andere der Reste $R_{2c}$ oder $R_{3c}$ ein Wasserstoffatom bedeuten, abgespalten wird oder

d) zur Herstellung von Verbindungen der allgemeinen Formel I, in der $R_1$ mit Ausnahme des Wasserstoffatoms die für $R_1$ in den Ansprüchen 1 bis 6 erwähnten Bedeutungen besitzt, eine Verbindung der allgemeinen Formel

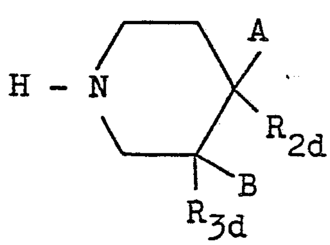
,(V)

in der
A und B wie in den Ansprüchen 1 bis 6 definiert sind, einer der Reste $R_{2d}$ oder $R_{3d}$ eine Gruppe der Formel

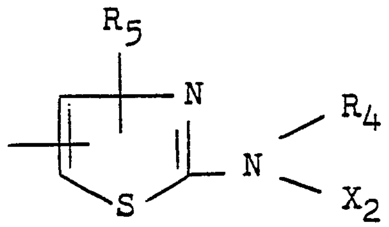

wobei $R_4$ und $R_5$ wie in den Ansprüchen 1 bis 6 definiert sind und
$X_2$ ein Wasserstoffatom oder einen Schutzrest, falls $R_4$ ein Wasserstoffatom oder einen der für $R_4$ in den Ansprüchen 1 bis 6 erwähnten Alkyl-oder gegebenenfalls substituierten Phenylalkylreste darstellt, und der andere der Reste $R_{2d}$ oder $R_{3d}$ ein Wasserstoffatom bedeuten, mit einer Verbindung der allgemeinen Formel

$Y_1$ -$R_1'''$ ,(VI)

in der
$R_1'''$ mit Ausnahme des Wasserstoffatoms die für $R_1$ erwähnten Bedeutungen besitzt und
$Y_1$ eine nukleophile Austrittsgruppe oder zusammen mit dem Wasserstoffatom des benachbarten Kohlenstoffatoms ein Sauerstoffatom bedeutet, umgesetzt und gegebenenfalls anschließend ein verwendeter Schutzrest abgespalten wird oder

e) zur Herstellung von Verbindungen der allgemeinen Formel I, in der A und B jeweils ein Wasserstoffatom darstellen, eine Verbindung der allgemeinen Formel

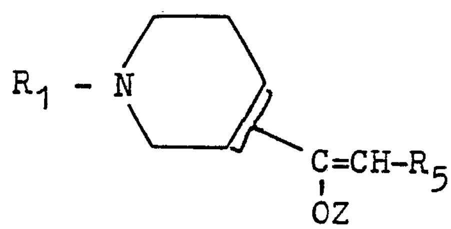

,(VII)

in der
$R_1$ und $R_5$ wie in den Ansprüchen 1 bis 6 definiert sind und
Z eine Trialkylsilylgruppe darstellt, mit Brom und anschließend mit einem Thioharnstoff der allgemeinen Formel

$H_2N$ - CS - NH - $R_4$ ,(VIII)

in der
$R_4$ wie in den Ansprüchen 1 bis 6 definiert ist, umgesetzt wird oder

f) zur Herstellung von Verbindungen der allgemeinen Formel I, in der $R_1$ eine gegebenenfalls durch eine Phenylgruppe substituierte Alkylgruppe mit 1 bis 3 Kohlenstoffatomen oder eine Alkylgruppe mit 4 bis 6 Kohlenstoffatomen und/oder $R_4$ ein Wasserstoffatom oder eine gegebenenfalls durch eine Phenylgruppe substituierte Alkylgruppe mit 1 bis 3 Kohlenstoffatomen bedeuten, wobei die bei der Definition der Reste $R_1$ und $R_4$ vorstehend erwähnten Phenylkerne jeweils durch ein Halogenatom, eine Methyl-oder Methoxygruppe substituiert sein können, eine Verbindung der allgemeinen Formel

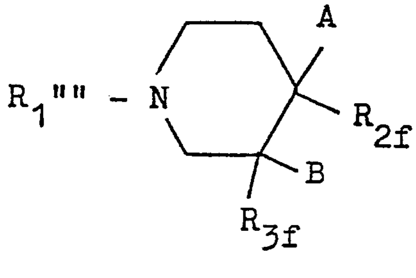

,(IX)

in der

A und B wie in den Ansprüchen 1 bis 6 definiert sind, einer der Reste $R_{2f}$ oder $R_{3f}$ eine Gruppe der Formel

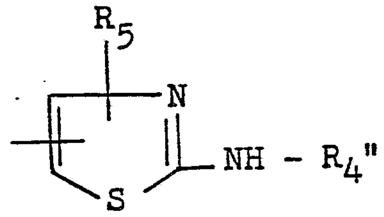

,

wobei $R_5$ wie in den Ansprüchen 1 bis 6 definiert ist,
$R_1''''$ und $R_4''$ die für $R_1$ bzw. $R_4$ erwähnten Bedeutungen besitzen, wobei jedoch mindestens in einem der Reste R1'''' oder $R_4''''$ die mit dem N-Atom verknüpfte Methylen gruppe des Restes $R_1''''$ oder $R_4''$ durch eine Carbonylgruppe ersetzt sein muß, und
der andere der Reste $R_{2f}$ oder $R_{3f}$ ein Wasserstoffatom bedeuten, reduziert wird oder

g) zur Herstellung von Verbindungen der allgemeinen Formel I, in der $R_4$ eine gegebenenfalls durch eine Phenylgruppe substituierte Alkylgruppe mit 1 bis 3 Kohlenstoffatomen darstellt, wobei der vorstehend erwähnte Phenylkern durch ein Halogenatom, eine Methyl-oder Methoxygruppe substituiert sein kann, ein Amin der allgemeinen Formel

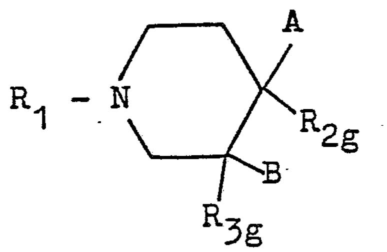

,(X)

in der
A, B und $R_1$ wie in den Ansprüchen 1 bis 6 definiert sind, einer der Reste $R_{2g}$ oder $R_{3g}$ eine Gruppe der Formel

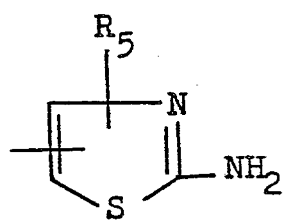

, wobei $R_5$ wie in den Ansprüchen 1 bis 6 definiert ist, und
der andere der Reste $R_{2g}$ oder $R_{3g}$ ein Wasserstoffatom bedeuten, mit einem Aldehyd der allgemeinen Formel

$Y_2$-$R_4'''$ ,(XI)

in der
$R_4'''$ eine gegebenenfalls durch eine Phenylgruppe substituierte Alkylgruppe mit 1 bis 3 Kohlenstoffatomen darstellt, wobei der vorstehend erwähnte Phenylring durch ein Halogenatom, eine Methyl-oder Methoxygruppe substituiert sein kann, und
$Y_2$ zusammen mit dem Wasserstoffatom des benachbarten Kohlenstoffatoms ein Sauerstoffatom bedeutet, reduktiv aminiert wird und
gewünschtenfalls anschließend eine so erhaltene Verbindung der allgemeinen Formel I, welche mindestens ein chirales Zentrum enthält, in ihre Enantiomeren aufgetrennt wird oder
eine so erhaltene Verbindung der allgemeinen Formel I in ihre Säureadditionssalze, insbesondere in ihre physiologisch verträglichen Säureadditionssalze mit organischen oder anorganischen Säuren übergeführt

wird.

Europäisches Patentamt

# EUROPÄISCHER RECHERCHENBERICHT

Nummer der Anmeldung

EP 88 10 1153

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | EP-A-0 244 018 (AKZO N.V.)<br>* Insgesamt *<br>----- | 1-12 | C 07 D 417/04<br>A 61 K 31/425<br>A 61 K 31/445 |
| | | | **RECHERCHIERTE SACHGEBIETE (Int. Cl.4)** |
| | | | C 07 D 417/00<br>A 61 K 31/00<br>C 07 D 417/04<br>A 61 K 31/425<br>A 61 K 31/445 |

Der vorliegende Recherchenbericht wurde für alle Patentansprüche erstellt

| Recherchenort | Abschlußdatum der Recherche | Prüfer |
|---|---|---|
| DEN HAAG | 13-05-1988 | DE BUYSER I.A.F. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)